(19) Europäisches Patentamt
European Patent Office
Office européen des brevets

(11) Veröffentlichungsnummer: **0 057 428**
**B1**

(12) # EUROPÄISCHE PATENTSCHRIFT

(45) Veröffentlichungstag der Patentschrift: **10.04.85**

(51) Int. Cl.⁴: **C 07 D 487/04**, A 61 K 31/55 // (C07D487/04, 243:00, 209:00)

(21) Anmeldenummer: **82100576.6**

(22) Anmeldetag: **28.01.82**

(54) **Tricyclische Pyrrole, Verfahren zu ihrer Herstellung, ihre Anwendung sowie sie enthaltende Arzneimittel.**

(30) Priorität: **02.02.81 CH 651/81**
**26.08.81 CH 5488/81**

(43) Veröffentlichungstag der Anmeldung:
**11.08.82 Patentblatt 82/32**

(45) Bekanntmachung des Hinweises auf die Patenterteilung:
**10.04.85 Patentblatt 85/15**

(84) Benannte Vertragsstaaten:
**AT BE CH DE FR GB IT LI LU NL SE**

(56) Entgegenhaltungen:
**EP-A-0 023 707**
**EP-A-0 024 582**
**DE-A-1 795 176**
**DE-A-2 050 344**

(73) Patentinhaber: **Byk Gulden Lomberg Chemische Fabrik GmbH, Byk-Gulden-Strasse 2, D-7750 Konstanz (DE)**

(72) Erfinder: **Senn-Bilfinger, Jörg, Dr., Renkenweg 12, D-7750 Konstanz (DE)**
Erfinder: **Riedel, Richard, Dr., Salmannsweilergasse 36, D-7750 Konstanz (DE)**
Erfinder: **Schaefer, Hartmann, Dr., Zum Purren 27, D-7750 Konstanz 16 (DE)**
Erfinder: **Klemm, Kurt, Prof. Dr., Im Weinberg 2, D-7753 Allensbach (DE)**

**EP 0 057 428 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Technisches Gebiet

Die Erfindung betrifft tricyclische Pyrrole, Verfahren zu ihrer Herstellung, ihre Anwendung sowie sie enthaltende Arzneimittel.

Die erfindungsgemässen Verbindungen werden in der pharmazeutischen Industrie als Zwischenprodukte und zur Herstellung von Medikamenten verwendet.

Stand der Technik

In der deutschen Auslegeschrift DE-AS 17 95 183 werden Pyridobenzodiazepin-6-on-derivate mit ulkushemmender, sekretionshemmender, antitussiver und teilweise antiemetischer Wirkung beansprucht, während in der DE-AS 16 20 523 Pyridobenzodiazepin-5-one mit rein antitussiver Wirkung beschrieben werden. In der japanischen Offenlegungsschrift 54-135788 werden Cyclopentabenzodiazepinone mit analgetischer und angstvermindernder Wirksamkeit beschrieben. In der deutschen Offenlegungsschrift DE-OS 20 50 344 werden weitere tricyclische Verbindungen beschrieben, die als Tranquilizer bzw. als Blutdrucksenker Verwendung finden sollen.

Darstellung der Erfindung

Gegenstand der Erfindung sind neue tricyclische Pyrrole der allgemeinen Formel I

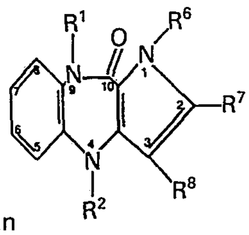

(I)

worin

- $R^1$ ein Wasserstoffatom oder einen Alkylrest mit 1 bis 4 Kohlenstoffatomen,
- $R^2$ die Gruppe $-CO-C_nH_{2n}-R^3$ oder ein Wasserstoffatom,
- $R^3$ ein Halogenatom oder die Gruppe $-N(R^4)R^5$,
- $R^4$ ein Wasserstoffatom, einen Alkylrest mit 1 bis 4 Kohlenstoffatomen oder einen Alkenylrest mit 3 bis 5 Kohlenstoffatomen und
- $R^5$ einen Alkylrest mit 1 bis 4 Kohlenstoffatomen, der gegebenenfalls durch eine Dialkylaminogruppe mit 1 bis 4 Kohlenstoffatomen in jedem Alkylrest substituiert ist, oder einen Alkenylrest mit 3 bis 5 Kohlenstoffatomen bedeutet, oder
- $R^4$ und $R^5$ gemeinsam und unter Einschluss des Stickstoffatoms, an das sie gebunden sind, einen Pyrrolidino-, Piperidino-, Morpholino-, Perhydroazepino- oder einen gegebenenfalls in 4-Position durch eine Methyl-, Ethyl- oder Benzylgruppe substituierten 1-Piperazinylrest oder einen gegebenenfalls in 4-Position durch eine Methylgruppe substituierten 1-Homo-piperazinylrest bedeuten,
- $R^6$, $R^7$ und $R^8$ gleich oder verschieden sind und Wasserstoff oder Alkyl mit 1 bis 4 Kohlenstoffatomen bedeuten und
- n 1 oder 2 darstellt,

sowie ihre Säureadditionssalze mit anorganischen und organischen Säuren.

Alkylreste mit 1 bis 4 Kohlenstoffatomen sind der Methyl-, Ethyl-, Propyl-, Butyl-, Isopropyl-, Isobutyl-, sek.-Butyl und tert.-Butylrest.

Als Alkenylreste mit 3 bis 5 Kohlenstoffatomen seien der Allylrest und der 2-Methylallylrest genannt.

Als Halogenatome seien das Chlor-, Brom- und Jodatom, bevorzugt das Chlor- und Bromatom, insbesondere das Chloratom genannt.

Als Salze kommen alle Säureadditionssalze in Betracht. Besonders erwähnt seien die pharmakologisch verträglichen Salze der in der Galenik üblicherweise verwendeten anorganischen und organischen Säuren. Pharmakologisch unverträgliche Salze, die beispielsweise bei der Herstellung der erfindungsgemässen Verbindungen im industriellen Massstab als Verfahrensproukte zunächst anfallen können, werden durch dem Fachmann bekannte Verfahren in pharmakologisch verträgliche Salze übergeführt. Als solche eignen sich beispielsweise wasserlösliche und wasserunlösliche Säureadditionssalze, wie das Hydrochlorid, Hydrobromid, Hydroiodid, Phosphat, Nitrat, Sulfat, Acetat, Citrat, Gluconat, Benzoat, Hibenzat (2-(4-Hydroxybenzoyl)-benzoat), Fendizoat (o-[(2'-Hydroxy-4-biphenylyl)-carbonyl]-benzoat), Butyrat, Sulfosalicylat, Maleat, Laurat, Malat, Fumarat, Succinat, Oxalat, Tartrat, Amsonat (4,4'-Diaminostilben-2,2'-disulfonat), Embonat (4,4'-Methylen-bis-3-hydroxy-2-naphthoat), Metembonat (4,4'-Methylen-bis-3-methoxy-2-naphthoat), Stearat, Tosilat (p-Toluolsulfonat), 2-Hydroxy-3-naphthoat, 3-Hydroxy-2-naphthoat, Mesilat (Methansulfonat).

Eine Ausgestaltung der Erfindung sind tricyclische Pyrrole der allgemeinen Formel $I^a$

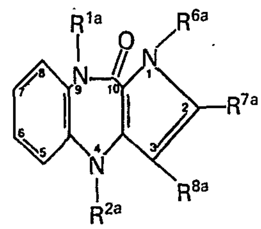

($I^a$)

worin

- $R^{1a}$ ein Wasserstoffatom oder einen Alkylrest mit 1 bis 4 Kohlenstoffatomen,
- $R^{2a}$ die Gruppe $-CO-C_{n^a}H_{2n^a}-R^{3a}$,
- $R^{3a}$ die Gruppe $-N(R^{4a})R^{5a}$,
- $R^{4a}$ ein Wasserstoffatom, einen Alkylrest mit 1 bis 4 Kohlenstoffatomen oder einen Alkenylrest mit 3 bis 5 Kohlenstoffatomen und
- $R^{5a}$ einen Alkylrest mit 1 bis 4 Kohlenstoffatomen, der gegebenenfalls durch eine Dialkylaminogruppe mit 1 bis 4 Kohlenstoffatomen in jedem Alkylrest substituiert ist, oder einen Alkenylrest mit 3 bis 5 Kohlenstoffatomen darstellt, oder
- $R^{4a}$ und $R^{5a}$ gemeinsam und unter Einschluss des Stickstoffatoms, an das sie gebunden sind, einen Pyrrolidino-, Piperidino-, Morpholino-, Perhydroazepino- oder einen gegebenenfalls

in 4-Position durch eine Methyl-, Ethyl- oder Benzylgruppe substituierten 1-Piperazinylrest oder einen gegebenenfalls in 4-Position durch eine Methylgruppe substituierten 1-Homo-piperazinylrest bedeuten,

$R^{6a}$, $R^{7a}$ und $R^{8a}$ gleich oder verschieden sind und Wasserstoff oder Alkyl mit 1 bis 4 Kohlenstoffatomen bedeuten und

$n^a$ 1 oder 2 darstellt,

sowie ihre Säureadditionssalze mit anorganischen und organischen Säuren.

Erwähnenswerte Vertreter der Ausgestaltung $I^a$ sind solche, in denen $R^{1a}$, $R^{6a}$, $R^{7a}$ und $R^{8a}$ gleich oder verschieden sind und Wasserstoff, Methyl oder Ethyl bedeuten, $R^{2a}$ und $R^{3a}$ die oben angegebene Bedeutung haben, $R^{4a}$ Wasserstoff, einen Alkylrest mit 1 bis 4 Kohlenstoffatomen oder einen Alkenylrest mit 3 Kohlenstoffatomen darstellt, $R^{5a}$ einen Alkylrest mit 1 bis 4 Kohlenstoffatomen, der gegebenenfalls endständig durch eine Dimethylamino- oder Diethylaminogruppe substituiert ist, oder einen Alkenylrest mit 3 Kohlenstoffatomen darstellt, oder $R^{4a}$ und $R^{5a}$ gemeinsam eine Pyrrolidino-, Piperidino-, Morpholino-, Piperazino- oder 4-Methyl- oder 4-Ethyl-1-piperazinylgruppe darstellen, und $n^a$ die Bedeutung 1 oder 2 hat, und ihre Säureadditionssalze.

Bevorzugte Vertreter der Ausgestaltung $I^a$ sind solche, in denen $R^{1a}$ Wasserstoff bedeutet, $R^{2a}$ und $R^{3a}$ die oben angegebene Bedeutung haben, $R^{4a}$ Wasserstoff oder einen Alkylrest mit 1 bis 4 Kohlenstoffatomen bedeutet, $R^{5a}$ einen Alkylrest mit 1 bis 4 Kohlenstoffatomen bedeutet, der gegebenenfalls endständig durch eine Dimethylaminogruppe substituiert ist, oder $R^{4a}$ und $R^{5a}$ gemeinsam eine Pyrrolidino-, Piperidino-, Morpholino-, Piperazino- oder 4-Methyl-1-piperazinylgruppe bedeuten, $R^{6a}$ Methyl, $R^{7a}$ Wasserstoff, $R^{8a}$ Methyl bedeutet und $n^a$ 1 oder 2 darstellt, und ihre pharmakologisch verträglichen Säureadditonssalze mit anorganischen und organischen Säuren.

Besonders bevorzugte Vertreter der Ausgestaltung $I^a$ sind solche, in denen $R^{1a}$ Wasserstoff bedeutet, $R^{2a}$ und $R^{3a}$ die oben angegebene Bedeutung haben, $R^{4a}$ Wasserstoff, Methyl, Ethyl oder Propyl bedeutet, $R^{5a}$ Methyl, Ethyl oder Propyl bedeutet oder $R^{4a}$ und $R^{5a}$ gemeinsam eine Pyrrolidino-, Piperidino- oder 4-Methyl-1-piperazinylgruppe bedeuten, $R^{6a}$ Methyl, $R^{7a}$ Wasserstoff, $R^{8a}$ Methyl und $n^a$ 1 darstellt, und ihre pharmakologisch verträglichen Säureadditionssalze mit anorganischen und organischen Säuren.

Eine weitere Ausgestaltung der Erfindung sind tricyclische Pyrrole der allgemeinen Formel $I^b$

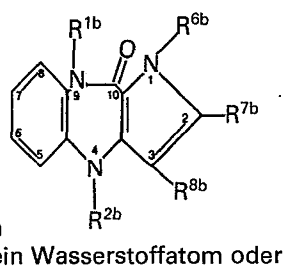

($I^b$)

worin

$R^{1b}$ ein Wasserstoffatom oder einen Alkylrest mit 1 bis 4 Kohlenstoffatomen,

$R^{2b}$ die Gruppe $-CO-C_{n^b}H_{2n^b}-R^{3b}$, und

$R^{3b}$ ein Halogenatom darstellt,

$R^{6b}$, $R^{7b}$ und $R^{8b}$ gleich oder verschieden sind und Wasserstoff oder Alkyl mit 1 bis 4 Kohlenstoffatomen bedeuten und

$n^b$ 1 oder 2 darstellt,

sowie ihre Säureadditionssalze mit anorganischen und organischen Säuren.

Erwähnenswerte Vertreter der Ausgestaltung $I^b$ sind solche, in denen $R^{1b}$, $R^{6b}$, $R^{7b}$ und $R^{8b}$ gleich oder verschieden sind und Wasserstoff, Methyl oder Ethyl bedeuten, $R^{2b}$ die oben angegebene Bedeutung hat, $R^{3b}$ Chlor oder Brom darstellt und $n^b$ die Bedeutung 1 oder 2 hat, und ihre Säureadditionssalze.

Bevorzugte Vertreter der Ausgestaltung $I^b$ sind solche, in denen $R^{1b}$ Wasserstoff bedeutet, $R^{2b}$ die oben angegebene Bedeutung hat, $R^{3b}$ Chlor oder Brom darstellt, $R^{6b}$ Methyl, $R^{7b}$ Wasserstoff, $R^{8b}$ Methyl und $n^b$ 1 oder 2 darstellt, und ihre Säureadditionssalze.

Besonders bevorzugte Vertreter der Ausgestaltung $I_b$ sind solche, in denen $R^{1b}$ Wasserstoff bedeutet, $R^{2b}$ die oben angegebene Bedeutung hat, $R^{3b}$ Chlor, $R^{6b}$ Methyl, $R^{7b}$ Wasserstoff, $R^{8b}$ Methyl und $n^b$ 1 darstellt, und ihre Säureadditionssalze.

Eine weitere Ausgestaltung der Erfindung sind tricyclische Pyrrole der allgemeinen Formel $I^c$

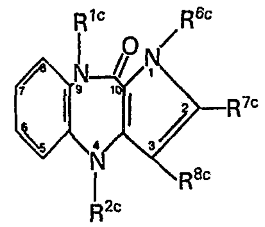

($I^c$)

worin

$R^{1c}$ ein Wasserstoffatom oder einen Alkylrest mit 1 bis 4 Kohlenstoffatomen,

$R^{2c}$ ein Wasserstoffatom darstellt und

$R^{6c}$, $R^{7c}$ und $R^{8c}$ gleich oder verschieden sind und Wasserstoff oder Alkyl mit 1 bis 4 Kohlenstoffatomen bedeuten,

sowie ihre Säureadditionssalze mit anorganischen und organischen Säuren.

Erwähnenswerte Vertreter der Ausgestaltung $I^c$ sind solche, in denen $R^{1c}$, $R^{6c}$, $R^{7c}$ und $R^{8c}$ gleich oder verschieden sind und Wasserstoff, Methyl oder Ethyl bedeuten, $R^{2c}$ die oben angegebene Bedeutung hat, und ihre Säureadditionssalze.

Bevorzugte Vertreter der Ausgestaltung $I^c$ sind solche, in denen $R^{1c}$ Wasserstoff bedeutet, $R^{2c}$ die oben angegebene Bedeutung hat, $R^{6c}$ Methyl, $R^{7c}$ Wasserstoff, $R^{8c}$ Methyl bedeutet, und ihre Säureadditionssalze.

Als Vertreter erfindungsgemässer Verbindungen seien beispielsweise genannt:

1,2,3,9-Tetramethyl-4-[(4-methyl-1-piperazinyl)-acetyl]-1,4,9,10-tetrahydropyrrolo[3.2-b][1.5]-benzodiazepin-10-on,

1,2,3-Triethyl-4-[(4-methyl-1-piperazinyl)-acetyl]-1,4,9,10-tetrahydropyrrolo[3.2-b][1.5]-benzodiazepin-10-on,

1,3-Dimethyl-4-piperidino-acetyl-1,4,9,10-tetra-

hydropyrrolo[3.2-b][1.5]benzodiazepin-10-on,

1,3-Dimethyl-4-[(4-methyl-1-piperazinyl)-acetyl]-1,4,9,10-tetrahydropyrrolo[3.2-b][1.5]-benzodiazepin-10-on,

1,3-Dimethyl-4-pyrrolidino-acetyl-1,4,9,10-tetrahydropyrrolo[3.2-b][1.5]benzodiazepin-10-on,

1-Ethyl-2,3-dimethyl-4-piperidino-acetyl-1,4,9,10-tetrahydropyrrolo[3.2-b][1.5]benzodiazepin-10-on,

1,3-Diethyl-9-methyl-4-[3-(4-methyl-1-piperazinyl)-propionyl]-1,4,9,10-tetrahydropyrrolo-[3.2-b][1.5]benzodiazepin-10-on,

1-Methyl-4-[3-(piperidino)-propionyl]-1,4,9,10-tetrahydropyrrolo[3.2-b][1.5]benzodiazepin-10-on,

1,9-Dibutyl-4-[(4-methyl-1-piperazinyl)-acetyl]-1,4,9,10-tetrahydropyrrolo[3.2-b][1.5]benzodiazepin-10-on,

1,2,3-Triethyl-4-morpholino-acetyl-1,4,9,10-tetrahydropyrrolo[3.2-b][1.5]benzodiazepin-10-on,

1-Isopropyl-2,9-dimethyl-4-piperidino-acetyl-1,4,9,10-tetrahydropyrrolo[3.2-b][1.5]benzodiazepin-10-on,

1-n-Butyl-2,3,9-trimethyl-4-perhydroazepino-acetyl-1,4,9,10-tetrahydropyrrolo[3.2-b][1.5]-benzodiazepin-10-on,

1,9-Di-n-butyl-4-[(4-methyl-1-piperazinyl)-acetyl]-1,4,9,10-tetrahydropyrrolo[3.2-b][1.5]-benzodiazepin-10-on,

2-Methyl-9-propyl-4-[2-(dimethylamino)-propionyl]-1,4,9,10-tetrahydropyrrolo[3.2-b][1.5]-benzodiazepin-10-on,

1,2,3-Trimethyl-4-[N-(2-dimethylaminoethyl)-N-methylamino]-acetyl-1,4,9,10-tetrahydropyrrolo-[3.2-b][1.5]benzodiazepin-10-on,

1,2,3-Trimethyl-4-[3-(dibutylamino)-propinyl]-1,4,9,10-tetrahydropyrrolo[3.2-b][1.5]benzodiazepin-10-on,

1,3,9-Triethyl-4-piperidino-acetyl-1,4,9,10-tetrahydropyrrolo[3.2-b][1.5]benzodiazepin-10-on,

1,3-Di-n-butyl-9-ethyl-2-methyl-4-[(4-methyl-1-piperazinyl)-acetyl]-1,4,9,10-tetrahydropyrrolo-[3.2-b][1.5]benzodiazepin-10-on,

9-tert.-Butyl-1,2-dimethyl-4-perhydroazepino-acetyl-1,4,9,10-tetrahydropyrrolo[3.2-b][1.5]-benzodiazepin-10-on,

1,3-Dimethyl-4-[2-(dimethylamino)-propionyl]-1,4,9,10-tetrahydropyrrolo[3.2-b][1.5]benzodiazepin-10-on,

1,3-Dimethyl-4-[N-(2-dimethylaminoethyl)-N-methylamino]-acetyl-1,4,9,10-tetrahydropyrrolo-[3.2-b][1.5]benzodiazepin-10-on,

9-n-Butyl-1,3-dimethyl-4-[(4-methyl-1-piperazinyl)-acetyl]-1,4,9,10-tetrahydropyrrolo-[3.2-b][1.5]benzodiazepin-10-on,

1,2,3-Trimethyl-4-[3-(4-methyl-1-piperazinyl)-propionyl]-1,4,9,10-tetrahydropyrrolo-[3.2-b][1.5]benzodiazepin-10-on,

1,2-Dimethyl-4-piperidino-acetyl-1,4,9,10-tetrahydropyrrolo[3.2-b][1.5]benzodiazepin-10-on,

1-Methyl-2-propyl-4-[(4-methyl-1-piperazinyl)-acetyl]-1,4,9,10-tetrahydropyrrolo[3.2-b][1.5]-benzodiazepin-10-on,

9-Isopropyl-1,3-dimethyl-4-piperidino-acetyl-1,4,9,10-tetrahydropyrrolo[3.2-b][1.5]benzodiazepin-10-on,

1,2,3-Trimethyl-4-piperidino-acetyl-1,4,9,10-tetrahydropyrrolo[3.2-b][1.5]benzodiazepin-10-on,

1,2,3-Trimethyl-4-[2-(4-methyl-1-piperazinyl)-propionyl]-1,4,9,10-tetrahydropyrrolo-[3.2-b][1.5]benzodiazepin-10-on,

9-Methyl-4-[(4-methyl-1-piperazinyl)-acetyl]-1,4,9,10-tetrahydropyrrolo[3.2-b][1.5]benzodiazepin-10-on,

9-Methyl-4-piperidino-acetyl-1,4,9,10-tetrahydropyrrolo[3.2-b][1.5]benzodiazepin-10-on,

1,3-Diethyl-4-[(4-methyl-1-piperazinyl)-acetyl]-1,4,9,10-tetrahydropyrrolo[3.2-b][1.5]benzodiazepin-10-on,

1,3-Diethyl-4-piperidino-acetyl-1,4,9,10-tetrahydropyrrolo[3.2-b][1.5]benzodiazepin-10-on,

1,2,3,9-Tetramethyl-4-piperidino-acetyl-1,4,9,10-tetrahydropyrrolo[3.2-b][1.5]benzodiazepin-10-on,

1,2,3,9-Tetramethyl-4-chloracetyl-1,4,9,10-tetrahydropyrrolo[3.2-b][1.5]benzodiazepin-10-on,

1,2,3-Triethyl-4-chloracetyl-1,4,9,10-tetrahydropyrrolo[3.2-b][1.5]benzodiazepin-10-on,

1,3-Dimethyl-4-chloracetyl-1,4,9,10-tetrahydropyrrolo[3.2-b][1.5]benzodiazepin-10-on,

1,3-Dimethyl-4-bromacetyl-1,4,9,10-tetrahydropyrrolo[3.2-b][1.5]benzodiazepin-10-on,

1-Ethyl-2,3-dimethyl-4-chloracetyl-1,4,9,10-tetrahydropyrrolo[3.2-b][1.5]benzodiazepin-10-on,

1,3-Diethyl-4-chloracetyl-1,4,9,10-tetrahydropyrrolo[3.2-b][1.5]benzodiazepin-10-on,

1-Methyl-4-bromacetyl-1,4,9,10-tetrahydropyrrolo[3.2-b][1.5]benzodiazepin-10-on,

1,9-Dibutyl-4-chloracetyl-1,4,9,10-tetrahydropyrrolo[3.2-b][1.5]benzodiazepin-10-on,

1-Isopropyl-2,9-dimethyl-4-chloracetyl-1,4,9,10-tetrahydropyrrolo[3.2-b][1.5]benzodiazepin-10-on,

1-n-Butyl-2,3,9-trimethyl-4-chloracetyl-1,4,9,10-tetrahydropyrrolo[3.2-b][1.5]benzodiazepin-10-on,

1,9-Di-n-butyl-4-chloracetyl-1,4,9,10-tetrahydropyrrolo[3.2-b][1.5]benzodiazepin-10-on,

2-Methyl-9-propyl-4-chloracetyl-1,4,9,10-tetrahydropyrrolo[3.2-b][1.5]benzodiazepin-10-on,

1,2,3-Trimethyl-4-chloracetyl-1,4,9,10-tetrahydropyrrolo[3.2-b][1.5]benzodiazepin-10-on,

1,3,9-Triethyl-4-bromacetyl-1,4,9,10-tetrahydropyrrolo[3.2-b][1.5]benzodiazepin-10-on,

1,3-Di-n-butyl-9-ethyl-2-methyl-4-chloracetyl-1,4,9,10-tetrahydropyrrolo[3.2-b][1.5]benzodiazepin-10-on,

9-tert.-Butyl-1,2-dimethyl-4-chloracetyl-1,4,9,10-tetrahydropyrrolo[3.2-b][1.5]benzodiazepin-10-on,

9-n-Butyl-1,3-dimethyl-4-bromacetyl-1,4,9,10-tetrahydropyrrolo[3.2-b][1.5]benzodiazepin-10-on,

1-Methyl-2-propyl-4-chloracetyl-1,4,9,10-tetrahydropyrrolo[3.2-b][1.5]benzodiazepin-10-on,

9-Isopropyl-1,3-dimethyl-4-chloracetyl-1,4,9,10-tetrahydropyrrolo[3.2-b][1.5]benzodiazepin-10-on,

9-Methyl-4-chloracetyl-1,4,9,10-tetrahydropyrrolo[3.2-b][1.5]benzodiazepin-10-on,

1,3-Diethyl-9-methyl-4-(3-chlor-propionyl)-1,4,9,10-tetrahydropyrrolo[3.2-b][1.5]benzodiazepin-10-on,

1-Methyl-4-(3-chlor-propionyl)-1,4,9,10-tetrahydropyrrolo[3.2-b][1.5]benzodiazepin-10-on,

2-Methyl-9-propyl-4-(2-chlor-propionyl)-1,4,9,10-tetrahydropyrrolo[3.2-b][1.5]benzodiazepin-10-on,

1,2,3-Trimethyl-4-(3-chlor-propionyl)-1,4,9,10-tetrahydropyrrolo[3.2-b][1.5]benzodiazepin-10-on,

1,3-Dimethyl-4-(2-chlor-propionyl)-1,4,9,10-tetrahydropyrrolo[3.2-b][1.5]benzodiazepin-10-on,

1,2,3-Trimethyl-4-(2-chlor-propionyl)-1,4,9,10-tetrahydropyrrolo[3.2-b][1.5]benzodiazepin-10-on,

1,3-Dimethyl-1,4,9,10-tetrahydropyrrolo-[3.2-b][1.5]benzodiazepin-10-on,

1-Ethyl-2,3-dimethyl-1,4,9,10-tetrahydropyrrolo[3.2-b][1.5]benzodiazepin-10-on,

1,3-Diethyl-1,4,9,10-tetrahydropyrrolo-[3.2-b][1.5]benzodiazepin-10-on,

1-Methyl-1,4,9,10-tetrahydropyrrolo-[3.2-b][1.5]benzodiazepin-10-on,

1,9-Dibutyl-1,4,9,10-tetrahydropyrrolo-[3.2-b][1.5]benzodiazepin-10-on,

1-Isopropyl-2,9-dimethyl-1,4,9,10-tetrahydropyrrolo[3.2-b][1.5]benzodiazepin-10-on,

1-n-Butyl-2,3,9-trimethyl-1,4,9,10-tetrahydropyrrolo[3.2-b][1.5]benzodiazepin-10-on,

1,9-Di-n-butyl-1,4,9,10-tetrahydropyrrolo-[3.2-b][1.5]benzodiazepin-10-on,

2-Methyl-9-propyl-1,4,9,10-tetrahydropyrrolo[3.2-b][1.5]benzodiazepin-10-on,

1,2,3-Trimethyl-1,4,9,10-tetrahydropyrrolo-[3.2-b][1.5]benzodiazepin-10-on,

1,3,9-Triethyl-1,4,9,10-tetrahydropyrrolo-[3.2-b][1.5]benzodiazepin-10-on,

1,3-Di-n-butyl-9-ethyl-2-methyl-1,4,9,10-tetrahydropyrrolo[3.2-b][1.5]benzodiazepin-10-on,

9-tert.-Butyl-1,2-dimethyl-1,4,9,10-tetrahydropyrrolo[3.2-b][1.5]benzodiazepin-10-on,

9-n-Butyl-1,3-dimethyl-1,4,9,10-tetrahydropyrrolo[3.2-b][1.5]benzodiazepin-10-on,

1-Methyl-2-propyl-1,4,9,10-tetrahydropyrrolo[3.2-b][1.5]benzodiazepin-10-on,

9-Isopropyl-1,3-dimethyl-1,4,9,10-tetrahydropyrrolo[3.2-b][1.5]benzodiazepin-10-on,

9-Methyl-1,4,9,10-tetrahydropyrrolo-[3.2-b][1.5]benzodiazepin-10-on,

1,3-Diethyl-9-methyl-1,4,9,10-tetrahydropyrrolo[3.2-b][1.5]benzodiazepin-10-on,

und ihre Säureadditionssalze, insbesondere 1,3-Dimethyl-4-pyrrolidinoacetyl-1,4,9,10-tetrahydropyrrolo[3.2-b][1.5]benzodiazepin-10-on, und 1,3-Dimethyl-4-[(4-methyl-1-piperazinyl)-acetyl]-1,4,9,10-tetrahydropyrrolo[3.2-b][1.5]benzodiazepin-10-on, und ihre pharmakologisch verträglichen Säureadditionssalze mit anorganischen und organischen Säuren.

Ein weiterer Gegenstand der Erfindung ist ein Verfahren zur Herstellung von tricyclischen Pyrrolen der allgemeinen Formel I, worin $R^1$, $R^2$, $R^6$, $R^7$ und $R^8$ die oben angegebenen Bedeutungen haben, sowie ihren Säureadditionssalzen mit anorganischen und organischen Säuren.

Das Verfahren ist dadurch gekennzeichnet, dass man Verbindungen der allgemeinen Formel VI

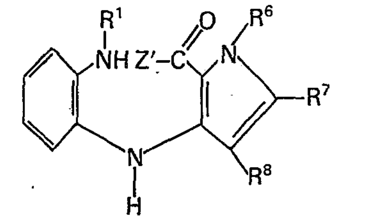

(VI)

worin $R^1$, $R^6$, $R^7$ und $R^8$ die oben angegebenen Bedeutungen haben und Z' eine geeignete Fluchtgruppe (= Abgangsgruppe) darstellt, cyclisiert und gegebenenfalls anschliessend bei den erhaltenen Tricyclen IV,

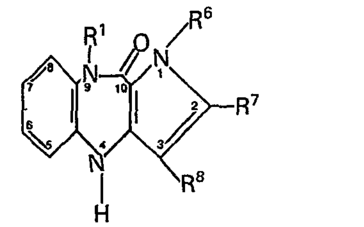

(IV)

worin $R^1$, $R^6$, $R^7$ und $R^8$ die oben angegebene Bedeutungen haben (= Verbindungen I mit $R^2$ = Wasserstoff), in 4-Position den Rest $-CO-C_nH_{2n}-R^3$, worin $R^3$ und n die oben angegebene Bedeutung haben, einführt, und dass man erhaltene Basen gegebenenfalls anschliessend in ihre Säureadditionssalze bzw. erhaltene Säureadditionssalze gegebenenfalls anschliessend in die freien Basen überführt.

Die Cyclisierung der Verbindung VI wird – je nach Art der Fluchtgruppe Z' – in an sich bekannter Weise durchgeführt. Eine geeignete Fluchtgruppe Z' ist eine Gruppe, die zusammen mit der Carbonylgruppe, an die sie gebunden ist, ein reaktives Carbonsäurederivat bildet. Eine geeignete Fluchtgruppe Z' ist beispielsweise eine Alkoxygruppe, eine Aminogruppe oder eine Hydroxygruppe.

Ist Z' z.B. eine Aminogruppe, so wird die Cyclisierung ohne oder in inerten bevorzugt polaren organischen Lösungsmitteln, wie niederen Alkoholen, z.B. Ethanol, gegebenenfalls in Gegenwart einer Säure, wie Halogenwasserstoffsäure, oder in Gegenwart einer Base, z.B. eines Alkalimetallalkanolats, bei Temperaturen zwischen 0°C und 200°C, bevorzugt bei der Siedetemperatur des verwendeten Lösungsmittels durchgeführt. Ist Z' eine Alkoxygruppe, so wird die Cyclisierung der Verbindungen VI bei Temperaturen zwischen 0°C und 200°C, vorzugsweise 20 und 160°C, ohne oder in Gegenwart eines inerten Lösungsmittels, gegebenenfalls in Gegenwart eines basischen oder vorzugsweise sauren Katalysators durchgeführt. Die Reaktionszeiten betragen 15 Minuten bis 6 Stunden. Als Lösungsmittel kommen beispielsweise Alkohole, wie Ethanol, Isopropanol oder Glykol; Ether, wie Dioxan oder Diphenylether; aromatische Kohlenwasserstoffe, wie Toluol, Xylol oder o-Dichlorbenzol; oder Dimethylsulfoxid in Frage. Als Katalysatoren seien genannt basische

Katalysatoren, wie Alkalimetallalkanolate, z. B. Natriummethylat oder Kalium-tert.-butanolat, oder vorzugsweise Natriumhydrid; oder saure Katalysatoren, wie organische oder anorganische Säuren, z. B. Essigsäure, Chloressigsäure, p-Toluolsulfonsäure, o-Chlorbenzoesäure, p-Tolylsäure, Nikotinsäure, Trifluoressigsäure, Fumarsäure, Chlorwasserstoffsäure, Benzoesäure oder Kaliumhydrogensulfat, vorzugsweise Phosphorsäure, wobei gegebenenfalls mehrere Mol saurer Katalysator je Mol Ausgangsverbindung eingesetzt werden.

Ist Z' eine Hydroxygruppe, so erfolgt die Cyclisierung der Verbindung VI beispielsweise in polaren Lösungsmitteln, bevorzugt unter saurer Katalyse, und vorteilhafterweise in Gegenwart eines Kondensationsmittels, wie Cyclohexylcarbodiimid, oder unter kontinuierlicher Abscheidung des bei der Reaktion entstehenden Wassers, z. B. durch azetropes Ausschleppen des Wassers am Wasserabscheider. Die Reaktion erfolgt bevorzugt bei Temperaturen zwischen 50°C und 200°C, insbesondere bei Temperaturen zwischen 100°C und 160°C.

Durch Cyclisierung der Verbindungen VI lassen sich Tricyclen IV, in denen $R^1$ ein Wasserstoffatom darstellt, aber auch Tricyclen IV, in denen $R^1$ einen Alkylrest mit 1 bis 4 Kohlenstoffatomen darstellt, synthetisieren. Zu Verbindungen IV, in denen $R^1$ einen Alkylrest darstellt, gelangt man in an sich bekannter Weise aber auch durch Deprotonierung und Alkylierung der entsprechenden Verbindungen IV, in denen $R^1$ ein Wasserstoffatom darstellt. Zur Deprotonierung der Verbindung IV werden geeignete Basen, z. B. Natriumhydrid, Natriumamid oder Kalium-t-butanolat, verwendet. Die Deprotonierung wird in inerten, wasserfreien, gegebenenfalls polaren Lösungsmitteln, wie Dimethylsulfoxid oder Dioxan, durchgeführt. Die anschliessende Alkylierung, beispielsweise mit Dialkylsulfaten, wie Dimethylsulfat, oder Alkylhalogeniden, wie Butyliodid, führt zu den gewünschten Tricyclen IV, in denen $R^1$ einen Alkylrest darstellt.

Die Einführung des Restes $-CO-C_nH_{2n}-R^3$ in den Tricyclus IV wird in an sich bekannter Weise vorgenommen:

Die Umsetzung der Tricyclen IV mit Verbindung V,

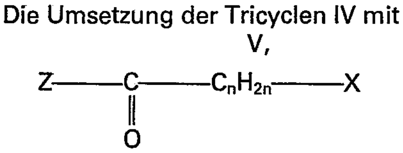

(V)

worin n die oben angegebene Bedeutung hat, X ein Halogenatom und Z eine Fluchtgruppe (= Abgangsgruppe) darstellt, führt zu Pyrrolen der allgemeinen Formel II,

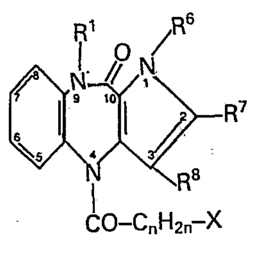

(II)

worin $R^1$, $R^6$, $R^7$, $R^8$ und n die oben angegebenen Bedeutungen haben und X ein Halogenatom darstellt (= Verbindungen der Formel I mit $R^2$ = $-CO-C_nH_{2n}-R^3$ und $R^3$ = Halogen).

Die sich gegebenenfalls anschliessende Umsetzung dieser Pyrolle mit Aminen III

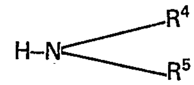

(III)

worin $R^4$ und $R^5$ die oben angegebenen Bedeutungen haben, liefert die Verbindungen I, worin $R^2$ die Gruppe $-CO-C_nH_{2n}-R^3$ und $R^3$ die Gruppe $-NR^4(R^5)$ darstellt und $R^4$ und $R^5$ die oben angegebene Bedeutung haben. Diese Verbindungen werden alternativ durch Reaktion der Tricyclen IV mit an sich bekannten Verbindungen III',

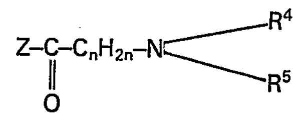

(III')

worin n, $R^4$ und $R^5$ die oben angegebenen Bedeutungen haben und Z eine Fluchtgruppe (= Abgangsgruppe) darstellt, erhalten.

Die Umsetzung der Tricyclen IV mit den Verbindungen V zu den Pyrrolen II erfolgt in an sich bekannter Weise.

Die Fluchtgruppe Z kann beispielsweise ein Halogenatom, insbesondere ein Chloratom, oder die Gruppe $X-C_nH_{2n}-CO-O-$ sein. Wenn Z ein Halogenatom ist, so wird die Reaktion vorteilhafterweise in Gegenwart eines säurebindenden Mittels (Protonenakzeptors) durchgeführt. Als geeignete Protonenakzeptoren seien beispielsweise genannt Alkalimetallcarbonate oder -bicarbonate, wie Natriumcarbonat oder Kaliumhydrogencarbonat; oder tertiäre organische Amine, wie Pyridin, Triethylamin oder Ethyldiisopropylamin.

Die Reaktion wird bevorzugt in inerten, wasserfreien Lösungsmitteln durchgeführt. Beispielsweise seien genannt: chlorierte Kohlenwasserstoffe, wie Methylenchlorid oder Chloroform; offenkettige oder cyclische Ether, wie Diethylether, Diethylenglycoldimethylether, Tetrahydrofuran oder insbesondere Dioxan; oder aromatische Kohlenwasserstoffe, wie Benzol oder Toluol.

Die Reaktionstemperatur, die von der Art der Fluchtgruppe, des Protonenakzeptors, der Reaktanten und des Lösungsmittels abhängig ist, liegt im allgemeinen zwischen 20°C und 150°C, insbesondere zwischen 50°C und 110°C, zum Beispiel bei der jeweiligen Siedetemperatur des verwendeten Lösungsmittels. Die Reaktionszeit schwankt zwischen 20 Minuten und 60 Stunden.

Die sich gegebenenfalls anschliessende Umsetzung der so erhaltenen Pyrrole II mit den Aminen III wird in an sich bekannter Weise und vorteilhafterweise in der Gegenwart eines Protonenakzeptors durchgeführt. Als solche eignen sich beispielsweise Alkalimetallcarbonate, wie Natriumcarbonat, oder Kaliumcarbonat, oder tertiäre Amine, wie Pyridin, Triethylamin oder Ethyldiisopropylamin.

Um Nebenreaktionen zu vermeiden, wird vorteilhafterweise auch ein Oberschuss an Verbindung III als Protonenakzeptor eingesetzt. In diesem Fall wird die Reaktion mit einem beispielsweise 2- bis 5fachen Überschuss an Verbindung III durchgeführt.

Die Reaktion wird in geeigneten, vorteilhafterweise inerten, wasserfreien Lösungsmitteln, wie niederen Alkoholen (z.B. Methanol, Ethanol oder Isopropanol) offenkettigen oder cyclischen Ethern (z.B. Tetrahydrofuran oder insbesondere Dioxan), aromatischen Kohlenwasserstoffen (z.B. Benzol oder insbesondere Toluol), oder chlorierten Kohlenwasserstoffen (z.B. Methylenchlorid) durchgeführt.

Die Reaktionstemperatur kann zwischen 0°C und 150°C liegen, wobei Temperaturen zwischen 50°C und 110°C – insbesondere die Siedetemperatur des verwendeten Lösungsmittels – bevorzugt sind. Die Reaktionszeit liegt – in Abhängigkeit vom eingesetzten Amin – zwischen einigen Minuten und mehreren Stunden; gegebenenfalls kann die Reaktion durch Zugabe eines Alkalimetalliodides beschleunigt werden.

Bei Verwendung leichtflüchtiger Amine wird die Reaktion bevorzugt bei niedrigeren Temperaturen oder in einer geschlossenen Apparatur durchgeführt.

Die Reaktion der Tricyclen IV mit den Verbindungen III' erfolgt ebenfalls in an sich bekannter Weise.

Die Fluchtgruppe Z ist eine Gruppe, die gemeinsam mit der Carbonylgruppe, an die sie gebunden ist, ein reaktives Carbonsäurederivat bildet. Als reaktive Carbonsäurederivate seien beispielsweise Säurehalogenide, -ester. -anhydride oder gemischte Anhydride, z.B. die aus der entsprechenden Säure (Z = OH) und Chlorameisensäureester oder Phosphoroxytrichlorid, genannt. Wenn Z ein Halogenatom ist, so wird die Reaktion vorteilhafterweise in Gegenwart eines säurebindenden Mittels (Protonenakzeptors) durchgeführt. Als geeignete Protonenakzeptoren seien beispielsweise genannt Alkalimetallcarbonate oder -bicarbonate, wie Natriumcarbonat oder Kaliumhydrogencarbonat; oder tertiäre organische Amine, wie Pyridin, Triethylamin oder Ethyldiisopropylamin.

Die Umsetzung der Pyrrole II mit den Aminen III ist das bevorzugte Verfahren zur Herstellung der Verbindungen I, in denen $R^2$ die Gruppe $-CO-C_nH_{2n}-R^3$ und $R^3$ die Gruppe $-NR^4(R^5)$ darstellt.

Säureadditionssalze erhält man durch Auflösen der freien Base in einem geeigneten Lösungsmittel, z.B. in einem chlorierten Kohlenwasserstoff, wie Methylenchlorid oder Chloroform, oder einem niedermolekularen aliphatischen Alkohol (Ethanol, Isopropanol), das die gewünschte Säure enthält, oder dem die gewünschte Säure anschliessend zugegeben wird.

Die Salze werden durch Filtrieren, Umfällen, Ausfällen mit einem Nichtlösungsmittel für das Anlagerungssalz oder durch Verdampfen des Lösungsmittels gewonnen.

Erhaltene Salze können durch Alkalisierung, z.B. mit wässrigem Natriumhydrogencarbonat, in die freien Basen umgewandelt werden, welche wiederum in Säureadditionssalze übergeführt werden können. Auf diese Weise lassen sich pharmakologisch nicht verträgliche Säureadditionssalze in pharmakologisch verträgliche Säureadditionssalze umwandeln.

Zu Verbindungen der Formel VI gelangt man durch Umsetzung von o-Halogennitrobenzolen VII mit 3-Aminopyrrolen VIII

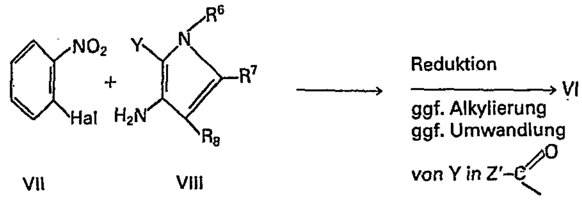


VII VIII

(wobei Hal ein Halogenatom – insbesondere ein Fluor- oder Chloratom – darstellt, Y eine

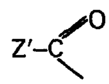

-Gruppe oder den Vorläufer einer

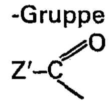

-Gruppe darstellt und $R^6$, $R^7$ und $R^8$ die oben angegebenen Bedeutungen haben), anschliessende Reduktion der Nitrogruppe zur Aminogruppe, gegebenenfalls anschliessende Umwandlung des Restes Y in einem geeigneten Rest

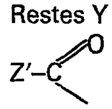

und/oder gegebenenfalls anschliessende Einführung des Alkylrestes $R^1$.

Unter dem Vorläufer einer

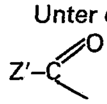

-Gruppe (= Y) versteht man einen Substituenten, der durch einen geeigneten, dem Fachmann geläufigen Verfahrensschnitt (z.B. Hydrolyse, Alkoholyse, Ansäuern, Verseifung etc.) in die

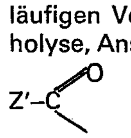

-Gruppe umgewandelt werden kann. Bevorzugte Vorläufer Y sind die Nitril-Gruppe (–CN) und die Carboxylat-Gruppe ($-COO^{\ominus}$), die durch Hydrolyse, Alkoholyse bzw. Ansäuern in die Amid-Gruppe, Ester-Gruppe bzw. in den Carbonsäurerest übergeführt werden.

Die Umsetzung der o-Halogennitrobenzole VII mit den 3-Aminopyrrolen VIII erfolgt in an sich bekannter Weise, bevorzugt unter Zusatz eines Deprotonierungsmittels, z.B. Natriumhydrid, Kaliumcarbonat oder eines tertiären Amins, in Lösungsmitteln wie Dimethylformamid, Dioxan, Tetrahydrofuran oder N-Methylpyrrolidon, je nach Art des Restes Hal in VII und je nach Art des verwendeten Deprotonierungsmittels bei Temperaturen zwischen 0°C und 150°C.

Die sich gegebenenfalls anschliessende Alkylierung erfolgt wie in der für die Tricyclen IV beschriebenen Weise.

3-Aminopyrrole der allgemeinen Formel VIII sind bekannt oder können nach bekannten Vorschriften analog hergestellt werden [Mu-Ill Lim, Robert S. Klein, Jack J. Fox, Tetrahedron Letters 21, 1013 (1980); Mu-Ill Lim, Robert S. Klein, Jack J. Fox, J. Org. Chem. 44, 3826 (1979)].

Die folgenden Beispiele erläutern die Erfindung näher, ohne sie einzuschränken. Die Abkürzung Schmp. bedeutet Schmelzpunkt, Kp. bedeutet Siedepunkt, Z. bedeutet Zersetzungspunkt. Unter «Ether» wird Diethylether verstanden. «Essigester» ist Essigsäureethylester.

Beispiele zur Ausführung der Erfindung

1. 3-Amino-1,4,5-trimethyl-1H-pyrrol-2-carbonsäureethylester

Eine Lösung von 23,2 g 1-Methyl-2-oxobutyronitril, 27,9 g (0,239 mol) Sarkosinethylester und 0,2 g p-Toluolsulfonsäure in 200 ml Chloroform wird 2 Stunden am Wasserabscheider gekocht bis sich im Kühler kein Wasser mehr abscheidet. Danach wird mit 20 ml gesättigter wässriger Natriumhydrogencarbonatlösung und mit 20 ml Wasser gewaschen. Nach dem Trocknen über Natriumsulfat wird am Rotationsverdampfer eingeengt, das zurückbleibende Öl zweimal in 100 ml trockenem Ethanol aufgenommen und das Lösungsmittel wiederum am Rotationsverdampfer im Vakuum abdestilliert. Das zurückbleibende Öl wird mit 300 ml 1 n Natriumethylat-/Ethanol-Lösung übergossen und 2 Stunden am Rückfluss erhitzt. Nach Abziehen des Lösungsmittels im Vakuum versetzt man den festen Rückstand mit 200 ml Eiswasser und extrahiert mehrmals mit Methylenchlorid. Beim Abziehen des Lösungsmittels verbleibt ein fester Rückstand, der aus Diisopropylether umkristallisiert wird. Ausbeute 14,3 g vom Schmp. 63 bis 64°C (Ethanol).

2. Kalium-(3-amino-1,4,5-trimethyl-1H-pyrrol-2-carboxylat)

36,6 g (0,187 mol) 3-Amino-1,4,5-trimethyl-1H-pyrrol-2-carbonsäureethylester und 11,4 g (0,213 mol) Kaliumhydroxid werden in 200 ml Methanol gelöst und 4 Stunden unter Rückfluss zum Sieden erhitzt. Die erhaltene Lösung wird zur Trockne eingeengt und ohne weitere Reinigung für die nächste Umsetzung verwendet.

3. Kalium-{1,4,5-trimethyl-3-[(2-nitrophenyl)-amino]-1H-pyrrol-2-carboxylat}

38 g 1-Chlor-2-nitrobenzol, 38,8 g Kalium-(3-amino-1,4,5-trimethyl-1H-pyrrol-2-carboxylat) (Rohprodukt aus Beispiel 2) und 36 g gemahlenes Kaliumcarbonat (Korngrösse 0,07 mm) werden in 120 ml 1-Methylpyrrolidon-(2) bei 140°C Innentemperatur unter Stickstoff gerührt. Nach 12 Stunden werden weitere 3,3 g 1-Chlor-2-nitrobenzol zugesetzt. Zur Aufarbeitung wird heiss filtriert und der Filterrückstand dreimal mit je 10 ml heissem (100°C) 1-Methyl-pyrrolidon-(2) nachgewaschen. Die vereinigten Filtrate werden ohne Reinigung weiterverarbeitet.

4. 3-[(2-Aminophenyl)-amino]-1,4,5-trimethyl-1H-pyrrol-2-carbonsäure

Die in Beispiel 3 erhaltene Lösung von 61 g Kalium{1,4,5-trimethyl-3-[(2-nitrophenyl)-amino]-1H-pyrrol-2-carboxylat} (0,19 mol) wird in einer Umlaufhydrierapparatur mit konzentrierter Salzsäure auf pH 2 eingestellt und mit 0,5 g Palladium-Kohle (10%ig) versetzt. Nachdem eine Innentemperatur von 40°C erreicht ist, wird mit der Hydrierung begonnen. Die Wasserstoffzugabe wird dabei so dosiert, dass die Innentemperatur bei etwa 100°C gehalten wird. Nachdem eine sprunghafte Abnahme der Wasserstoffaufnahme beobachtet wird, lässt man die Lösung abkühlen, filtriert sie unter Stickstoff und wäscht den Filterrückstand sechsmal mit je 5 ml 1-Methylpyrrolidon-(2). Am Rotationsverdampfer werden die vereinigten Filtrate auf die Hälfte ihres Volumens eingeengt. Die erhaltene Lösung wird ohne weitere Reinigung in Beispiel 5 weiterverarbeitet.

5. 1,2,3-Trimethyl-1,4,9,10-tetrahydropyrrolo-[3.2-b][1.5]benzodiazepin-10-on

Die in Beispiel 4 erhaltene, 49,2 g (0,19 mol) 3-[(2-Aminophenyl)-amino]-1,4,5-trimethyl-1H-pyrrol-2-carbonsäure enthaltende Lösung wird mit 15 ml konzentrierter Salzsäure versetzt und 12 Stunden auf eine Innentemperatur von 140°C erhitzt. Dabei wird entstehendes Wasser kontinuierlich abdestilliert. Zur Aufarbeitung wird das Lösungsmittel am Rotationsverdampfer im Wasserstrahlvakuum so weit wie möglich abdestilliert. Der Rückstand wird mit 100 ml Wasser aufgenommen und bei 80°C mit konzentrierter Salzsäure auf pH 2 eingestellt. Man kühlt unter Rühren auf 0°C ab. Der entstandene Niederschlag wird abgesaugt. Das Filtrat wird dreimal mit je 50 ml Essigsäureethylester extrahiert. Die vereinigten Filtrate werden mit Natriumsulfat getrocknet, eingeengt und der Rückstand wiederum mit 50 ml Wasser aufgenommen und bei 80°C mit konzentrierter Salzsäure auf pH 2 eingestellt. Nach Abkühlen im Eisbad auf 0°C wird abgesaugt. Die vereinigten Filterrückstände werden mit Wasser gewaschen und aus Essigsäureethylester umkristallisiert.

6. 4-Chloracetyl-1,2,3-trimethyl-1,4,9,10-tetrahydropyrrolo[3.2-b][1.5]benzodiazepin-10-on

Zu 31 g 1,2,3-Trimethyl-1,4,9,10-tetrahydropyrrolo[3.2-b][1.5]benzodiazepin-10-on, 6 g wasserfreiem Kaliumcarbonat, 40 ml Dioxan und 20 ml Toluol tropft man in 30 Minuten bei 70 bis 80°C eine Lösung von 21 g Chloracetylchlorid in 10 ml Toluol und rührt noch 2 Stunden bei dieser Temperatur. Man engt zur Trockne ein, kocht den Rückstand dreimal mit je 25 ml Chloroform aus und engt das Filtrat zur Trockne ein. Man erhält 3,6 g der Titelverbindung.

7. 1,2,3-Trimethyl-4-[(4-methyl-1-piperazinyl)-acetyl]-1,4,9,10-tetrahydropyrrolo[3.2-b][1.5]-benzodiazepin-10-on

289 mg 4-Chloracetyl-1,2,3-trimethyl-1,4,9,10-tetrahydropyrrolo[3.2-b][1.5]benzodiazepin-10-on, 200 mg N-Methylpiperazin und 10 ml trockenes Dioxan werden 2,5 Stunden auf 70 bis 80°C erwärmt. Man versetzt mit 10 ml gesättigter wässriger Natriumhydrogencarbonat-Lösung und extrahiert mehrmals mit Essigester. Die vereinigten Extrakte werden über Natriumsulfat getrocknet und im Vakuum eingeengt. Das resultierende Öl wird in wenig Dioxan/Acetonitril aufgenommen und zur Kristallisation gebracht.

8. 3-Amino-1,4,5-trimethyl-1H-pyrrol-2-carbonitril

Zur Suspension von 7,8 g (0,74 mol) N-Methylaminoacetonitrilhydrochlorid in 70 ml Chloroform werden nacheinander 10,3 ml (0,74 mol) Triethylamin, 7,2 g 3-Cyanobutanon-2 (0,74 mol) und 0,5 g p-Toluolsulfonsäure zugesetzt und das Gemisch 5 Stunden am Wasserabscheider gekocht. Nach dem Abkühlen wird mit je 10 ml Wasser zweimal gewaschen, über Natriumsulfat getrocknet und das Lösungsmittel im Vakuum abgezogen. Zur vollständigen Entfernung des Chloroforms wird zweimal in je 100 ml trockenem Ethanol aufgenommen und wiederum im Vakuum eingeengt. Das resultierende Öl wird 2 Stunden in 100 ml 1 n Natriumethylat/Ethanol erwärmt (50°C Badtemperatur), das Ethanol teilweise abgezogen, in 100 ml Wasser aufgenommen und mehrmals mit Methylenchlorid extrahiert. Die Extrakte liefern nach dem Trocknen über Natriumsulfat und Einengen am Rotationsverdampfer einen festen Rückstand, der aus Diisopropylether umkristallisiert wird.

9. 3-Amino-1,5-dimethyl-1H-pyrrol-2-carbonsäureethylester

Analog Beispiel 1 werden aus 8,3 g (0,1 mol) 2-Oxobutyronitril und 11,7 g (0,1 mol) Sarkosinethylester 5,5 g der Titelverbindung erhalten.

10. 1,5-Dimethyl-3-(2-nitrophenyl)-amino-1H-pyrrol-2-carbonsäureethylester

Zur Lösung von 18,2 g (0,1 mol) 3-Amino-1,5-dimethyl-1H-pyrrol-2-carbonsäureethylester in 200 ml trockenem Dimethylformamid werden bei 0°C unter einer Inertgasatmosphäre ($N_2$) 6,0 g (0,2 mol) käufliches 80%iges Natriumhydrid (in Paraffin) addiert. Nach 1 Stunde ist die Gasentwicklung abgeklungen. Innerhalb von 30 Minuten werden 28 g (0,2 mol) 2-Fluornitrobenzol zugetropft. Nach 3 Stunden lässt man auf Zimmertemperatur kommen, giesst auf 100 g Eis und extrahiert mehrmals mit Ethylacetat. Die vereinigten Extrakte werden mit Wasser gewaschen, über Natriumsulfat getrocknet und das Lösungsmittel im Vakuum abgezogen. Das resultierende gelborange Öl kristallisiert nach kurzer Zeit durch. Ausbeute 22,5 g.

11. 3-(2-Aminophenyl)-amino-1,5-dimethyl-1H-pyrrol-2-carbonsäureethylester

Eine Lösung von 3,0 g (0,01 mol) 1,5-Dimethyl-3-(2-nitrophenyl)-amino-1H-pyrrol-2-carbonsäureethylester in 50 ml Ethylacetat wird nach Zusatz einer katalytischen Menge Palladium/Kohle in einer Umlaufhydrierapparatur unter geringem Oberdruck bei Raumtemperatur bis zur theoretischen Wasserstoffaufnahme hydriert. Nach dem Abfiltrieren des Katalysators wird im Vakuum eingeengt und der kristalline Rückstand aus wenig Isopropanol umkristallisiert. Ausbeute 2,1 g.

12. 1,2-Dimethyl-1,4,9,10-tetrahydro-pyrrolo[3.2-b][1.5]benzodiazepin-10-on

2,73 g (0,01 mol) 3-(2-Aminophenyl)-amino-1,5-dimethyl-1H-pyrrol-2-carbonsäureethylester werden 2 Stunden in einer Lösung von 0,25 g (0,011 mol) Natrium in 40 ml trockenem Ethanol am Rückfluss gekocht. Nach dem Abkühlen wird mit Essigsäure vorsichtig neutralisiert, das Lösungsmittel im Vakuum abgezogen und der gelbliche Rückstand aus Dioxan umkristallisiert. Ausbeute 1,9 g.

13. 4-Chloracetyl-1,2-dimethyl-1,4,9,10-tetrahydro-pyrrolo[3.2-b][1.5]benzodiazepin-10-on

Analog Beispiel 6 werden aus 2,3 g (0,01 mol) 1,2-Dimethyl-1,4,9,10-tetrahydro-pyrrolo-[3.2-b][1.5]benzodiazepin-10-on und 1,6 ml (0,02 mol) Chloracetylchlorid nach Reinigung an neutralem Kieselgel (Methylenchlorid) 1,5 g der Titelverbindung erhalten.

14. 1,2-Dimethyl-4-[(4-methylpiperazin-1-yl)-acetyl]-1,4,9,10-tetrahydro-pyrrolo[3.2-b]-[1.5]benzodiazepin-10-on

Eine Lösung von 303 mg (0,001 mol) 4-Chloracetyl-1,2-dimethyl-1,4,9,10-tetrahydro-pyrrolo[3.2-b][1.5]benzodiazepin-10-on und 200 mg (0,002 mol) N-Methylpiperazin in 10 ml trockenem Dioxan wird 2 Stunden am Rückfluss gekocht. Man versetzt mit 20 ml gesättigter wässriger Natriumhydrogencarbonat-Lösung und extrahiert mehrmals mit Essigester. Die vereinigten Extrakte werden über Natriumsulfat getrocknet und im Vakuum eingeengt. Das resultierende Öl wird an neutralem Kieselgel chromatographiert (Methylenchlorid/Methanol). Ausbeute: 280 mg.

15. 1,2-Dimethyl-4-piperidinoacetyl-1,4,9,10-tetrahydro-pyrrolo[3.2-b][1.5]benzodiazepin-10-on

Analog Beispiel 14 werden aus 606 mg (0,002 mol) 4-Chloracetyl-1,2-dimethyl-1,4,9,10-tetrahydro-pyrrolo[3.2-b][1.5]benzodiazepin-10-on und 340 mg Piperidin 420 mg der Titelverbindung gewonnen.

16. 4-Dimethylaminoacetyl-1,2-dimethyl-1,4,9,10-tetrahydro-pyrrolo[3.2-b][1.5]benzodiazepin-10-on

350 mg der Titelverbindung werden analog Beispiel 14 aus 606 mg (0,002 mol) 4-Chloracetyl-1,2-dimethyl-1,4,9,10-tetrahydro-pyrrolo[3.2-b][1.5]-benzodiazepin-10-on und 2 ml einer gesättigten alkoholischen Dimethylamin-Lösung erhalten.

17. 1,2,9-Trimethyl-1,4,9,10-tetrahydro-pyrrolo[3.2-b][1.5]benzodiazepin-10-on

Zu einer 5°C kalten Lösung von 2,3 g (0,01 mol) 1,2-Dimethyl-1,4,9,10-tetrahydro-pyrrolo[3.2-b][1.5]benzodiazepin-10-on in 20 ml trockenem Dimethylsulfoxid und 20 ml Tetrahydrofuran wer-

den unter einer Inertgasatmosphäre ($N_2$) 0,33 g (0,011 mol) 80%iges Natriumhydrid (in Paraffin) addiert. Man rührt 30 Minuten und lässt auf Raumtemperatur kommen (30 Minuten). Danach wird wiederum auf 5°C abgekühlt und 1,4 g (0,01 mol) Methyliodid, gelöst in 5 ml trockenem Tetrahydrofuran, werden zugetropft. Nach 2 Stunden Rühren bei 35°C werden 50 ml Eiswasser addiert und mehrmals mit Essigester extrahiert. Die gesammelten Extrakte werden nach dem Einengen im Vakuum an neutralem Kieselgel chromatographiert (Methylenchlorid/Methanol), Ausbeute: 1,7 g.

18. 1,2,9-Trimethyl-4-[(4-methylpiperazin-1-yl)-acetyl]-1,4,9,10-tetrahydro-pyrrolo[3.2-b][1.5]-benzodiazepin-10-on

Analog Beispiel 14 werden aus 317 mg (0,001 mol) 4-Chloracetyl-1,2,9-trimethyl-1,4,9,10-tetrahydro-pyrrolo[3.2-b][1.5]benzodiazepin-10-on (hergestellt analog Beispiel 6) und 200 mg (0,002 mol) N-Methylpiperazin 270 mg der Titelverbindung erhalten.

19. 3-Amino-1,4-dimethyl-1H-pyrrol-2-carbonsäureethylester

9,1 g N-(2-Cyan-1-propenyl)-sarkosinethylester (hergestellt durch Umsetzung von Methacrylnitril mit Brom und anschliessende Reaktion mit Sarkosinethylester) werden in 50 ml wasserfreiem Dimethylformamid gelöst und unter Rühren bei 20°C mit 300 mg 80%igem Natriumhydrid versetzt. Nach Erwärmen des Reaktionsgemisches auf 30°C setzt eine exotherme Reaktion ein (Temperaturanstieg auf 45°C), die nach 30 Minuten beendet ist. Man erhält eine Lösung der Titelverbindung (Kp. 70°C, 1,3 Pa), die ohne Aufarbeitung sofort weiter umgesetzt wird.

20. 1,4-Dimethyl-3-(2-nitrophenyl)-amino-1H-pyrrol-2-carbonsäureethylester

Die nach Beispiel 19 erhaltene Lösung von 3-Amino-1,4-dimethyl-1H-pyrrol-2-carbonsäureethylester in Dimethylformamid wird auf 0°C abgekühlt und unter Rühren mit 3,0 g 80%igem Natriumhydrid und 14,1 g 2-Fluornitrobenzol versetzt. Die nach Entfernung des Kühlbades bei gleichzeitigem Temperaturanstieg auf 40°C zu beobachtende Gasentwicklung ist nach 3 Stunden beendet. Man rührt über Nacht, kühlt auf 0°C ab und versetzt mit 100 g Eis und 100 ml Wasser. Der entstandene Niederschlag wird abgesaugt und mit kaltem (0°C) Methanol gut gewaschen. Man erhält 13,5 g (87%) der Titelverbindung vom Schmp. 114°C (aus Ethanol).

21. 1,4-Dimethyl-3-(2-nitrophenyl)-amino-1H-pyrrol-2-carbonsäuremethylester

Analog zu der in Beispiel 20 beschriebenen Arbeitsweise erhält man durch Umsetzung von 3-Amino-1,4-dimethyl-1H-pyrrol-2-carbonsäuremethylester mit 2-Chlornitrobenzol die Titelverbindung vom Schmp. 110 bis 113°C (aus Isopropanol).

22. 3-(2-Aminophenyl)-amino-1,4-dimethyl-1H-pyrrol-2-carbonsäureethylester

Zu einer auf 50°C erwärmten Lösung von 80 g 1,4-Dimethyl-3-(2-nitrophenyl)-amino-1H-pyrrol-2-carbonsäureethylester und 30 ml Hydrazinhydrat in 2 l Ethanol gibt man eine ethanolische Suspension von Raney-Nickel in solchen Portionen, dass eine Temperatur von 70°C nicht überschritten wird. Nach 45 Minuten beobachtet man bei weiterer Raney-Nickel-Zugabe keine Stickstoffentwicklung mehr. Das Raney-Nickel wird noch heiss abfiltriert und mit Ethanol gewaschen. Nach dem Einengen des Filtrats erhält man 58 g (82%) der Titelverbindung vom Schmp. 90°C (aus Isopropanol).

23. 1,3-Dimethyl-1,4,9,10-tetrahydropyrrolo-[3.2-b][1.5]benzodiazepin-10-on

10 g 3-(2-Aminophenyl)-amino-1,4-dimethyl-1H-pyrrol-2-carbonsäureethylester werden mit 30 ml 85%iger Phosphorsäure in einem Kolben gut vermischt und im Wasserstrahlvakuum auf 90°C erhitzt. Nach 15 Minuten lässt man abkühlen, versetzt mit 50 g Eis und 100 ml Wasser und saugt den ausgefallenen gelben Niederschlag ab. Man erhält 8,1 g (98%) der Titelverbindung vom Schmp. 184°C (aus Isopropanol).

24. 4-Chloracetyl-1,3-dimethyl-1,4,9,10-tetrahydro-pyrrolo[3.2-b][1.5]benzodiazepin-10-on

Zu 1,9 g 1,3-Dimethyl-1,4,9,10-tetrahydropyrrolo[3.2-b][1.5]benzodiazepin-10-on und 5,5 g gepulvertem wasserfreien Kaliumcarbonat in 50 ml Dioxan tropft man innerhalb von 10 Minuten unter Rühren bei 50°C eine Lösung von 1,47 g Chloracetylchlorid in 20 ml Dioxan. Nach mehrstündigem Rühren saugt man ab und engt das Filtrat zur Trockne ein. Man erhält 1,75 g (69%) der Titelverbindung vom Schmp. 267 bis 269°C (Acetonitril).

25. 1,3-Dimethyl-4-[(4-methyl-1-piperazinyl)-acetyl]-1,4,9,10-tetrahydropyrrolo[3.2-b][1.5]-benzodiazepin-10-on

Eine Lösung von 1,75 g 4-Chloracetyl-1,3-dimethyl-1,4,9,10-tetrahydro-pyrrolo[3.2-b][1.5]-benzodiazepin-10-on und 1,15 g N-Methylpiperazin in 50 ml wasserfreiem Dioxan wird 5 Stunden unter Rühren auf 50°C erhitzt. Nach dem Abkühlen saugt man vom ausgefallenen Niederschlag ab, engt das Filtrat ein, versetzt den Rückstand mit 50 ml gesättigter Natriumhydrogencarbonatlösung und extrahiert mehrmals mit Essigsäureethylester. Nach dem Trocknen über Natriumsulfat und dem Einengen erhält man 1,5 g der Titelverbindung vom Schmp. 205 bis 207°C (aus Essigsäureethylester). Das Dihydrochlorid schmilzt bei 212°C (Z., aus Wasser). Das Hemifumarat schmilzt bei 173°C (aus Ethanol).

26. 1,3-Dimethyl-4-piperidinoacetyl-1,4,9,10-tetrahydro-pyrrolo[3.2-b][1.5]benzodiazepin-10-on

Durch Umsetzung von 4-Chloracetyl-1,3-dimethyl-1,4,9,10-tetrahydro-pyrrolo[3.2-b][1.5]-benzodiazepin-10-on mit Piperidin erhält man analog Beispiel 25 die Titelverbindung vom

Schmp. 198°C (aus Isopropanol). Das Hydrochlorid schmilzt bei 285°C (Z., aus Dioxan).

27. 1,3-Dimethyl-4-pyrrolidinoacetyl-1,4,9,10-tetrahydro-pyrrolo[3.2-b][1.5]benzodiazepin-10-on

Durch Umsetzung von 4-Chloracetyl-1,3-dimethyl-1,4,9,10-tetrahydro-pyrrolo[3.2-b][1.5]-benzodiazepin-10-on mit Pyrrolidin erhält man analog Beispiel 25 die Titelverbindung vom Schmp. 209 bis 212°C (aus Isopropanol). Das Hydrochlorid schmilzt bei 218 bis 220°C (aus Isopropanol).

28. 1,3-Dimethyl-4-[N-(2-dimethylaminoethyl)-aminoacetyl]-1,4,9,10-tetrahydro-pyrrolo-[3.2-b][1.5]benzodiazepin-10-on

Durch Umsetzung von 4-Chloracetyl-1,3-dimethyl-1,4,9,10-tetrahydro-pyrrolo[3.2-b][1.5]-benzodiazepin-10-on mit N,N-Dimethylethylendiamin erhält man analog Beispiel 25 die Titelverbindung vom Schmp. 176 bis 180°C (aus Acetonitril).

29. 1,3-Dimethyl-4-morpholinoacetyl-1,4,9,10-tetrahydropyrrolo[3.2-b][1.5]benzodiazepin-10-on

Durch Umsetzung von 4-Chloracetyl-1,3-dimethyl-1,4,9,10-tetrahydropyrrolo[3.2-b][1.5]-benzodiazepin-10-on mit Morpholin erhält man analog Beipiel 25 die Titelverbindung vom Schmp. 208 bis 210°C (aus Ethanol).

30. 1,3-Dimethyl-4-(1-piperazinyl)-acetyl-1,4,9,10-tetrahydropyrrolo[3.2-b][1.5]benzodiazepin-10-on

Durch Umsetzung von 4-Chloracetyl-1,3-dimethyl-1,4,9,10-tetrahydropyrrolo[3.2-b][1.5]-benzodiazepin-10-on mit Piperazin erhält man analog Beispiel 25 die Titelverbindung vom Schmp. 205 bis 208°C (aus Ethanol).

31. 4-Ethylaminoacetyl-1,3-dimethyl-1,4,9,10-tetrahydropyrrolo[3.2-b][1.5]benzodiazepin-10-on

Durch Einleiten von Ethylamin in eine siedende ethanolische Lösung von 4-Chloracetyl-1,3-dimethyl-1,4,9,10-tetrahydropyrrolo[3.2-b][1.5]-benzodiazepin-10-on (2,0 g) erhält man 1,5 g (75%) der Titelverbindung vom Schmp. 175 bis 176°C (aus Acetonitril).

32. 1,3-Dimethyl-4-di-n-propylaminoacetyl-1,4,9,10-tetrahydropyrrolo[3.2-b][1.5]benzodiazepin-10-on

Durch Umsetzung von 4-Chloracetyl-1,3-dimethyl-1,4,9,10-tetrahydropyrrolo[3.2-b][1.5]-benzodiazepin-10-on mit Di-n-propylamin bei 80°C erhält man analog Beispiel 25 die Titelverbindung vom Schmp. 176 bis 177°C (aus Acetonitril).

33. 1,3-Dimethyl-4-dimethylaminoacetyl-1,4,9,10-tetrahydropyrrolo[3.2-b][1.5]benzodiazepin-10-on

Analog Beispiel 25 erhält man die Titelverbindung vom Schmp. 219 bis 220°C (aus Isopropanol) durch Umsetzung von 4-Chloracetyl-1,3-dimethyl-1,4,9,10-tetrahydropyrrolo[3.2-b][1.5]-benzodiazepin-10-on mit überschüssigem Dimethylamin bei Raumtemperatur.

34. 4-Diethylaminoacetyl-1,3-dimethyl-1,4,9,10-tetrahydropyrrolo[3.2-b][1.5]benzodiazepin-10-on

Durch Umsetzung von 4-Chloracetyl-1,3-dimethyl-1,4,9,10-tetrahydropyrrolo[3.2-b][1.5]-benzodiazepin-10-on mit Diethylamin bei 80°C erhält man analog Beispiel 25 die Titelverbindung vom Schmp. 174 bis 175°C (aus Isopropanol).

35. 4-(3-Brompropionyl)-1,3-dimethyl-1,4,9,10-tetrahydropyrrolo[3.2-b][1.5]benzodiazepin-10-on

Zu 4,54 g 1,3-Dimethyl-1,4,9,10-tetrahydropyrrolo[3.2-b][1.5]benzodiazepin-10-on und 5,0 g gepulvertem wasserfreiem Kaliumcarbonat in 80 ml trockenem Dioxan tropft man bei 50 bis 60°C 3,42 g 3-Brompropionylchlorid in 20 ml Dioxan innerhalb von 10 Minuten ein und rührt noch 2 Stunden bei der gleichen Temperatur. Das Lösungsmittel wird im Vakuum abgezogen, der feste Rückstand mit 300 ml Eiswasser verrührt und abgesaugt. Man erhält nach dem Umkristallisieren aus Isopropanol 5,1 g der Titelverbindung vom Schmp. 194 bis 195°C (Z.)

36. 4-(2-Brompropionyl)-1,3-dimethyl-1,4,9,10-tetrahydropyrrolo[3.2-b][1.5]benzodiazepin-10-on

Durch Umsetzung von 1,3-Dimethyl-1,4,9,10-tetrahydropyrrolo[3.2-b][1.5]benzodiazepin-10-on mit 2-Brompropionylchlorid erhält man die Titelverbindung analog Beispiel 35 vom Schmp. 245 bis 246°C (Z., aus Isopropanol).

37. 1,3-Dimethyl-4-[3-(4-methyl-piperazin-1-yl)propionyl]-1,4,9,10-tetrahydropyrrolo[3.2-b][1.5]benzodiazepin-10-on

1,5 g 4-(3-Brompropionyl)-1,3-dimethyl-1,4,9,10-tetrahydropyrrolo[3.2-b][1.5]benzodiazepin-10-on und 0,85 g N-Methylpiperazin werden in 50 ml Dioxan 15 Stunden bei 80°C erhitzt. Danach wird im Vakuum bis zur Trockne eingeengt, mit gesättigter Sodalösung (20 ml) verrührt und dreimal mit je 30 ml Ethylacetat extrahiert. Die vereinigten Extrakte werden über Natriumsulfat getrocknet und das Lösungsmittel im Vakuum abgezogen. Der feste Rückstand wird an Kieselgel (neutral) mit einem Dichlormethan-Methanol-Gemisch (95/05) als Elutionsmittel chromatographiert. Man erhält 1,2 g der Titelverbindung vom Schmp. 187°C (aus Isopropanol).

38. 1,3-Dimethyl-4-(3-pyrrolidino-propionyl)-1,4,9,10-tetrahydropyrrolo[3.2-b][1.5]benzodiazepin-10-on

1,9 g 4-(3-Brompropionyl)-1,3-dimethyl-1,4,9,10-tetrahydropyrrolo[3.2-b][1.5]benzodiazepin-10-on und 0,75 g Pyrrolidin werden in 50 ml Dioxan 4 Stunden bei 80°C erhitzt. Nach dem Abziehen des Lösungsmittels im Vakuum

wird in wenig gesättigter Sodalösung aufgenommen, dreimal mit je 30 ml Ethylacetat extrahiert, die vereinigten Extrakte über Natriumsulfat getrocknet und das Lösungsmittel im Vakuum abgezogen. Der verbleibende kristalline Rückstand wird aus wenig Acetonitril umkristallisiert. Ausbeute: 1,5 g der Titelverbindung vom Schmp. 185°C.

39. 1,3-Dimethyl-4-(3-dimethylamino-propionyl)-1,4,9,10-tetrahydropyrrolo[3.2-b][1.5]benzodiazepin-10-on

Durch Umsetzung von 4-(3-Brompropionyl)-1,3-dimethyl-1,4,9,10-tetrahydropyrrolo[3.2-b][1.5]-benzodiazepin-10-on mit Dimethylamin erhält man analog Beispiel 38 die Titelverbindung vom Schmp. 175°C (aus Acetonitril).

40. 1,3-Dimethyl-4-(2-pyrrolidino-propionyl)-1,4,9,10-tetrahydropyrrolo[3.2-b][1.5]benzodiazepin-10-on

Durch Umsetzung von 4-(2-Brompropionyl)-1,3-dimethyl-1,4,9,10-tetrahydropyrrolo[3.2-b][1.5]-benzodiazepin-10-on mit Pyrrolidin erhält man analog Beispiel 38 die Titelverbindung vom Schmp. 213–215°C (aus Isopropanol).

41. 1,3-Dimethyl-4-[2-(4-methyl-piperazin-1-yl)propionyl]-1,4,9,10-tetrahydropyrrolo[3.2-b][1.5]benzodiazepin-10-on

Durch Umsetzung von 4-(2-Brompropionyl)-1,3-dimethyl-1,4,9,10-tetrahydropyrrolo[3.2-b][1.5]-benzodiazepin-10-on mit N-Methylpiperazin erhält man analog Beispiel 38 die Titelverbindung vom Schmp. 204 bis 205°C (aus Acetonitril).

42. 1,3-Dimethyl-4-[(4-methyl-1-piperazinyl)-acetyl]-1,4,9,10-tetrahydropyrrolo[3.2-b][1.5]-benzodiazepin-10-on

Man erwärmt ein Gemisch von 1,00 g (4-Methylpiperazin-1-yl)-essigsäure und 0,20 g 75%igem Natriumhydrid (in Paraffinöl) in 16 ml Dimethylformamid bei 50 bis 80°C so lange, bis die Wasserstoffentwicklung beendet ist (2 bis 3 Stunden). Zu dem gebildeten Natriumsalz der Säure gibt man 1,40 g 1,3-Dimethyl-1,4,9,10-tetrahydropyrrolo[3.2-b][1.5]benzodiazepin-10-on und tropft bei −10°C 0,99 g 98% Phosphoroxichlorid in 10 Minuten zu. Man rührt 4 Stunden bei −10°C, 4 Stunden bei 0°C und 20 Stunden bei Raumtemperatur. Man giesst den Ansatz auf Eis, stellt mit Natronlauge auf pH 3,5, schüttelt mit Methylenchlorid aus, stellt die wässrige Phase auf pH 9 und schüttelt wieder mit Methylenchlorid aus. Man wäscht die organische Phase mit Wasser und engt sie im Vakuum ein. Man erhält 0,62 g der Titelverbindung vom Schmp. 205 bis 207°C (aus Essigsäureethylester).

43. 1,3-Dimethyl-4-pyrrolidinoacetyl-1,4,9,10-tetrahydropyrrolo[3.2-b][1.5]benzodiazepin-10-on

Zu einer Suspension von 1,29 g Pyrrolidin-1-yl-essigsäure in 20 ml Tetrahydrofuran werden bei 0°C 1,1 g Chlorameisensäureethylester zugetropft. Man fügt 2,28 g 1,3-Dimethyl-1,4,9,10-tetrahydropyrrolo[3.2-b][1.5]benzodiazepin-10-on zu der entstandenen Suspension, rührt noch eine Stunde bei 0°C und anschliessend 4 Stunden bei Raumtemperatur. Man giesst auf 160 ml 2 n Natronlauge, extrahiert mit Toluol und engt die organische Phase zur Trockne ein. Nach säulenchromatographischer Reinigung (Kieselgel; Dioxan/Methanol 1:1) erhält man 2,1 g der Titelverbindung vom Schmp. 210 bis 212°C (aus Isopropanol).

Gewerbliche Anwendbarkeit

Die tricyclischen Pyrrole der allgemeinen Formel I bzw. die der Ausgestaltung $I^a$, $I^b$ und $I^c$ und ihre Säureadditionssalze besitzen wertvolle Eigenschaften, die sie gewerblich verwertbar machen.

Die tricyclischen Pyrrole der allgemeinen Formel I, worin $R^2$ die Gruppe $-CO-C_nH_{2n}-R^3$ und $R^3$ die Gruppe $-N(R^4)R^5$ darstellt, und worin $R^1$, $R^4$, $R^5$, $R^6$, $R^7$, $R^8$ und n die oben angegebenen Bedeutungen haben, sowie die Pyrrole der allgemeinen Formel $I^a$ und ihre Säureadditionssalze sind erfindungsgemässe pharmakologisch wirksame Verbindungen, die durch eine ausgezeichnete Magen- und Darmschutzwirkung bei Warmblütern gekennzeichnet sind. Sie hemmen insbesondere die Magen- und Darmgeschwürbildung.

Ferner weisen sie, bedingt durch eine geringe Toxität und das Fehlen wesentlicher Nebenwirkungen, eine günstige therapeutische Breite auf. Im übrigen haben die Verbindungen nur eine geringe anticholinerge Wirkung, was sich beispielsweise an der geringen Hemmung der durch Carbachol ausgelösten Salivation feststellen lässt.

Die erfindungsgemässen tricyclischen Pyrrole der allgemeinen Formel I, worin $R^2$ ein Wasserstoffatom oder die Gruppe $-CO-C_nH_{2n}-R^3$ darstellt und $R^3$ ein Halogenatom ist, und die Pyrrole der allgemeinen Formeln $I^b$ und $I^c$ sind wertvolle Zwischenprodukte für die Herstellung der pharmakologisch wirksamen Verbindungen. Die ausgezeichnete Wirksamkeit der pharmakologisch wirksamen tricyclischen Pyrrole und ihrer pharmakologisch verträglichen Säureadditionssalze ermöglicht ihren Einsatz in der Human- oder Veterinärmedizin, wobei sie zur Behandlung und Prophylaxe von Krankheiten, die auf Erkrankungen des Magens und Darms beruhen, verwendet werden. Beispielsweise werden akuter und chronischer Ulcus ventriculi und Ulcus duodeni, Gastritis, hyperacider Reizmagen und medikamentös bedingte Magenbeschwerden bei Mensch und Tier behandelt.

Ein weiterer Gegenstand der Erfindung ist daher ein Verfahren zur Behandlung von Säugetieren, die an einer der obengenannten Krankheiten erkrankt sind. Das Verfahren ist dadurch gekennzeichnet, dass man dem erkrankten Säugetier eine therapeutisch wirksame und pharmakologisch verträgliche Menge einer oder mehrerer der oben genannten pharmakologisch wirksamen Verbindungen verabreicht.

Gegenstand der Erfindung sind ausserdem die hier genannten pharmakologisch wirksamen Verbindungen zur Anwendung in diesem Verfahren.

Ebenso umfasst die Erfindung die Verwendung der pharmakologisch wirksamen Verbindungen bei der Herstellung von Arzneimitteln, die zur Bekämpfung der angeführten Krankheiten eingesetzt werden.

Ein weiterer Gegenstand der Erfindung sind Arzneimittel, die ein oder mehrere tricyclische Pyrrole der allgemeinen Formel $I^a$, worin $R^{1a}$, $R^{2a}$, $R^{6a}$, $R^{7a}$ und $R^{8a}$ die oben angegebenen Bedeutungen haben, und/oder ihre pharmakologisch verträglichen Säureadditionssalze mit anorganischen und organischen Säuren enthalten.

Die Arzneimittel werden nach an sich bekannten Verfahren hergestellt. Als Arzneimittel werden die erfindungsgemässen, pharmakologisch wirksamen Verbindungen (= Wirkstoffe) entweder als solche oder vorzugsweise in Kombination mit geeigneten pharmazeutischen Trägerstoffen in Form von Tabletten, Dragées, Kapseln, Suppositorien, Emulsionen, Suspensionen oder Lösungen eingesetzt. Als Trägerstoffe kommen hierbei z.B. Lösungsmittel, Vermittler für die Arzneimittelaufnahme durch den Körper, Formulierungshilfsmittel, Süssungsmittel, Geschmackskorrigenzien, Farbstoffe oder Konservierungsmittel in Frage. Enthalten die pharmazeutischen Zubereitungen neben den erfindungsgemässen Verbindungen pharmazeutische Trägerstoffe, so beträgt der Wirkstoffgehalt dieser Mischung 1 bis 95, vorzugsweise 10 bis 85 Gewichtsprozent der Gesamtmischung. Die Tagesdosis liegt in der Humanmedizin bei oraler Gabe im allgemeinen zwischen etwa 0,3 und 150, vorzugsweise 1,5 und 75, insbesondere 3 und 15 mg/kg Körpergewicht.

Sollen die erfindungsgemässen, pharmakologisch wirksamen Verbindungen und/oder ihre Säureadditionssalze zur Behandlung der oben genannten Krankheiten eingesetzt werden, so können die pharmazeutischen Zubereitungen auch einen oder mehrere andere pharmakologisch aktive Bestandteile anderer Arzneimittelgruppen, wie Antacida, beispielsweise Aluminiumhydroxid, Magnesiumaluminat; Tranquilizer, wie Benzodiazepine, beispielsweise Diazepam; Spasmolytika, wie z.B. Bietamiverin, Camylofin; Anticholinergica, wie z.B. Oxyphencyclimin, Phencarbamid; Lokalanaesthetika, wie z.B. Tetracain, Procain; gegebenenfalls auch Fermente, Vitamine, Aminosäuren etc. enthalten.

Die Formulierung der Wirkstoffe kann z.B. auf folgende Weise erfolgen:

a) Tabletten mit 20 mg Wirkstoff

10 kg 1,3-Dimethyl-4-[(4-methyl-1-piperazinyl)-acetyl]-1,4,9,10-tetrahydropyrrolo[3.2-b][1.5]-benzodiazepin-10-on, 45 kg Milchzucker, 31 kg Maisstärke und 3 kg Polyvinylpyrrolidon werden mit ca. 20 Liter Wasser befeuchtet und durch ein Sieb mit 1,25 mm Maschenweite granuliert. Das Granulat wird im Wirbelschichttrockner bis zu einer Feuchte von 50 bis 60% getrocknet und dann mit 8 kg Natriumcarboxymethylcellulose, 2 kg Talkum und 1 kg Magnesiumstearat versetzt. Das fertige Granulat wird zu Tabletten à 200 mg und 8 mm Durchmesser gepresst.

b) Kapseln mit einem Wirkstoffgehalt von 15 mg

150 g 1,3-Dimethyl-4-pyrrolidino-acetyl-1,4,9,10-tetrahydropyrrolo[3.2-b][1.5]benzodiazepin-10-on, 845 g mikrokristalline Cellulose und 5 g amorphe Kieselsäure werden feingepulvert, gut vermischt und in Hartgelatinekapseln Grösse 4 abgefüllt.

Pharmakologie

Die ausgezeichnete Magenschutzwirkung der pharmakologisch wirksamen tricyclischen Pyrrole lässt sich am Modell der sogenannten Shay-Ratte nachweisen. Hierbei erweisen sich die erfindungsgemässen Verbindungen dem bekannten Handelsprodukt Carbenoxolon in der Magenschutzwirkung und der therapeutischen Breite eindeutig überlegen. Die untersuchten Verbindungen sind wie folgt mit laufenden Nummern versehen worden:

| Lfd. Nr. | Name der Verbindung |
|---|---|
| 1 | Carbenoxolon |
| 2 | 1,3-Dimethyl-4-[(4-methyl-1-piperazinyl)-acetyl]-1,4,9,10-tetrahydropyrrolo-[3.2-b][1.5]benzodiazepin-10-on |
| 3 | 1,3-Dimethyl-4-piperidinoacetyl-1,4,9,10-tetrahydropyrrolo[3.2-b][1.5]benzodiazepin-10-on |
| 4 | 1,3-Dimethyl-4-pyrrolidinoacetyl-1,4,9,10-tetrahydropyrrolo[3.2-b][1.5]benzodiazepin-10-on |
| 5 | 4-Diethylaminoacetyl-1,3-dimethyl-1,4,9,10-tetrahydropyrrolo[3.2-b][1.5]benzodiazepin-10-on |
| 6 | 1,3-Dimethyl-4-dimethylaminoacetyl-1,4,9,10-tetrahydropyrrolo[3.2-b][1.5]benzodiazepin-10-on |
| 7 | 1,3-Dimethyl-4-di-n-propylaminoacetyl-1,4,9,10-tetrahydropyrrolo[3.2-b][1.5]benzodiazepin-10-on |
| 8 | 4-Ethylaminoacetyl-1,3-dimethyl-1,4,9,10-tetrahydropyrrolo[3.2-b][1.5]benzodiazepin-10-on |
| 9 | 1,3-Dimethyl-4-(3-pyrrolidino-propionyl)-1,4,9,10-tetrahydropyrrolo[3.2-b][1.5]benzodiazepin-10-on |
| 10 | 1,3-Dimethyl-4-(3-dimethylamino-propionyl)-1,4,9,10-tetrahydropyrrolo-[3.2-b][1.5]benzodiazepin-10-on |

Der Einfluss der erfindungsgemässen Verbindungen auf die durch Pylorusligatur und 100 mg/kg Acetylsalicylsäure ausgelöste Magengeschwürbildung bei der Ratte und auf die Magensaftsekretion der Ratte wird in der folgenden Tabelle dargestellt:

Antiulcerogene Wirkung und Toxizität von tricyclischen Pyrrolen

| Lfd. Nr. | Toxizität Maus i.v. $LD_{50}$ mg/kg | Magenschutzwirkung Ratte p.o. $ED_{50}$ mg/kg | TQ $LD_{50}$/$ED_{50}$ | Magensekretionshemmung+) Ratte % |
|---|---|---|---|---|
| 1 | 290 | 70 | 4,1 | 7 |
| 2 | 100 | 1,4 | 71,4 | 25 |
| 3 | 95 | 4,5 | 21,1 | 3 |
| 4 | 100 | 2,2 | 45,5 | 0 |
| 5 | 105 | 6,0 | 17,5 | 20 |
| 6 | | 6,5 | | 10 |
| 7 | 110 | 6,5 | 16,9 | 10 |
| 8 | 140 | 5,5 | 25,5 | 20 |
| 9 | 70 | 6,0 | 11,7 | 25 |
| 10 | 120 | 4,5 | 26,7 | 21 |

$ED_{50}$ = Dosis, die den Ulcusindex bei der behandelten Gruppe gegenüber der Kontrollgruppe um 50% mindert

$LD_{50}$ = Dosis, bei der 50% der Tiere sterben

TQ = Therapeutischer Quotient $LD_{50}/ED_{50}$
+) % Hemmung = Hemmung der Magensekretion (in %) 4 Stunden nach Applikation der antiulcerogenen $ED_{50}$

Es sei besonders darauf hingewiesen, dass bei Verbindung 1 zwar eine $ED_{50}$ noch bestimmbar ist, die Dosiswirkungskurve dann jedoch sehr stark abflacht, so dass selbst bei 300 mg/kg keine wesentliche Steigerung der antiulcerogenen Wirkung erreichbar ist. Im Gegensatz dazu ist die Wirkung der erfindungsgemässen Verbindungen streng dosisabhängig; es lassen sich Hemmeffekte bis 99% (30 mg/kg) erreichen.

Die Prüfung der antiulcerogenen Wirkung erfolgte nach der Methode der sogenannten Shay-Ratte:

Die Ulcusprovokation erfolgt bei 24 Stunden nüchtern gehaltenen Ratten (weiblich, 180–200 g, 4 Tiere je Käfig auf hohem Gitterrost) durch Pylorusligatur (unter Diethylethernarkose) und orale Applikation von 100 mg/10 ml/kg Acetylsalicylsäure. Die zu testenden Substanzen werden oral (10 ml/kg) 1 Stunde vor der Pylorusligatur verabreicht. Der Wundverschluss wird mittels Michelklammern vorgenommen. 4 Stunden danach erfolgt die Tötung der Tiere im Etherrausch durch Atlas-Dislokation und die Resektion des Magens. Der Magen wird längs eröffnet und auf einer Korkplatte fixiert, nachdem zuvor die Menge des sezernierten Magensaftes (Volumen) bestimmt wurde; mit einem Stereomikroskop werden bei 10facher Vergrösserung Anzahl und Grösse (= Durchmesser) vorhandener Ulcera ermittelt. Das Produkt aus Schweregrad (gemäss nachfolgender Punkteskala) und Anzahl der Ulcera dient als individueller Ulcusindex.

Punkteskala:

| | | |
|---|---|---|
| keine Ulcera | | 0 |
| Ulcusdurchmesser | 0,1–1,4 mm | 1 |
| | 1,5–2,4 mm | 2 |
| | 2,5–3,4 mm | 3 |
| | 3,5–4,4 mm | 4 |
| | 4,5–5,4 mm | 5 |
| | >5,5 mm | 6 |

Als Mass für den antiulcerogenen Effekt dient die Minderung des mittleren Ulcus-Index jeder behandelten Gruppe gegenüber dem der Kontrollgruppe (= 100%). Die $ED_{50}$ bezeichnet die Dosis, die den mittleren Ulcusindex um 50% mindert.

Bestimmung der Toxizität

Die Toxizitätsuntersuchungen werden an weiblichen NMRI-Mäusen (Körpergewicht 23–30 g) durchgeführt. Die Tiere (5 Tiere pro Dosis) erhalten Futter und Wasser ad libitum. Verschiedene Dosen der Substanzen werden intravenös (Injektionsdauer 1 Minute) verabreicht. Die Beobachtungsdauer beträgt 7 Tage. Die $LD_{50}$, d.h. die Dosis, bei der 50% der Tiere sterben, wird mittels linearer Regression ermittelt.

**Patentansprüche für die Vertragsstaaten BE CH DE FR GB IT LI LU NL SE**

1. Tricyclische Pyrrole der allgemeinen Formel I

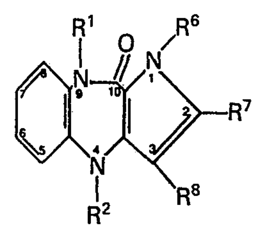

(I)

worin

$R^1$ ein Wasserstoffatom oder einen Alkylrest mit 1 bis 4 Kohlenstoffatomen,

$R^2$ die Gruppe $-CO-C_nH_{2n}-R^3$ oder ein Wasserstoffatom,

$R^3$ ein Halogenatom oder die Gruppe $-N(R^4)R^5$,

$R^4$ ein Wasserstoffatom, einen Alkylrest mit 1 bis 4 Kohlenstoffatomen oder einen Alkenylrest mit 3 bis 5 Kohlenstoffatomen und

$R^5$ einen Alkylrest mit 1 bis 4 Kohlenstoffatomen, der gegebenenfalls durch eine Dialkylaminogruppe mit 1 bis 4 Kohlenstoffatomen in jedem Alkylrest substituiert ist, oder einen Alkenylrest mit 3 bis 5 Kohlenstoffatomen bedeutet, oder

$R^4$ und $R^5$ gemeinsam und unter Einschluss des Stickstoffatoms, an das sie gebunden sind, einen Pyrrolidino-, Piperidino-, Morpholino-, Perhydroazepino- oder einen gegebenenfalls in 4-Position durch eine Methyl-, Ethyl- oder Benzylgruppe substituierten 1-Piperazinylrest oder einen gegebenenfalls in 4-Position durch eine Methylgruppe substituierten 1-Homopiperazinylrest bedeuten,

$R^6$, $R^7$ und $R^8$ gleich oder verschieden sind und Wasserstoff oder Alkyl mit 1 bis 4 Kohlenstoffatomen bedeuten und

n 1 oder 2 darstellt,

und ihre Säureadditionssalze mit anorganischen und organischen Säuren.

2. Tricyclische Pyrrole nach Anspruch 1, gekennzeichnet durch die allgemeine Formel $I^a$

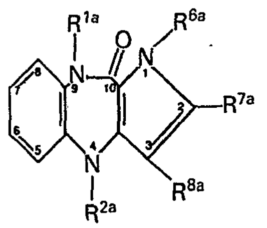

($I^a$)

worin

$R^{1a}$ ein Wasserstoffatom oder einen Alkylrest mit 1 bis 4 Kohlenstoffatomen,

$R^{2a}$ die Gruppe $-CO-C_{n^a}H_{2n^a}-R^{3a}$,

$R^{3a}$ die Gruppe $-N(R^{4a})R^{5a}$,

$R^{4a}$ ein Wasserstoffatom, einen Alkylrest mit 1 bis 4 Kohlenstoffatomen oder einen Alkenylrest mit 3 bis 5 Kohlenstoffatomen und

$R^{5a}$ einen Alkylrest mit 1 bis 4 Kohlenstoffatomen, der gegebenenfalls durch eine Dialkylaminogruppe mit 1 bis 4 Kohlenstoffatomen in jedem Alkylrest substituiert ist, oder einen Alkenylrest mit 3 bis 5 Kohlenstoffatomen darstellt, oder

$R^{4a}$ und $R^{5a}$ gemeinsam und unter Einschluss des Stickstoffatoms, an das sie gebunden sind, einen Pyrrolidino-, Piperidino-, Morpholino-, Perhydroazepino- oder einen gegebenenfalls in 4-Position durch eine Methyl-, Ethyl- oder Benzylgruppe substituierten 1-Piperazinylrest oder einen gegebenenfalls in 4-Position durch eine Methylgruppe substituierten 1-Homopiperazinylrest bedeuten,

$R^{6a}$, $R^{7a}$ und $R^{8a}$ gleich oder verschieden sind und Wasserstoff oder Alkyl mit 1 bis 4 Kohlenstoffatomen bedeuten und

$n^a$ 1 oder 2 darstellt,

und ihre Säureadditionssalze mit anorganischen und organischen Säuren.

3. Tricyclische Pyrrole der allgemeinen Formel $I^a$ nach Anspruch 2, in denen $R^{1a}$ Wasserstoff bedeutet, $R^{2a}$ und $R^{3a}$ die in Anspruch 2 angegebene Bedeutung haben, $R^{4a}$ Wasserstoff oder einen Alkylrest mit 1 bis 4 Kohlenstoffatomen bedeutet, $R^{5a}$ einen Alkylrest mit 1 bis 4 Kohlenstoffatomen bedeutet, der gegebenenfalls endständig durch eine Dimethylaminogruppe substituiert ist, oder $R^{4a}$ und $R^{5a}$ gemeinsam eine Pyrrolidino-, Piperidino-, Morpholino-, Piperazino- oder 4-Methyl-1-piperazinylgruppe bedeuten, $R^{6a}$ Methyl, $R^{7a}$ Wasserstoff, $R^{8a}$ Methyl bedeutet und $n^a$ 1 oder 2 darstellt, und ihre pharmakologisch verträglichen Säureadditionssalze mit anorganischen und organischen Säuren.

4. Tricyclische Pyrrole nach Anspruch 1, gekennzeichnet durch die allgemeine Formel $I^b$

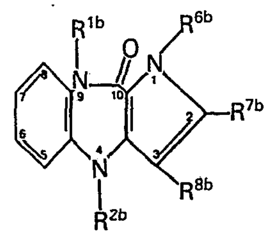

($I^b$)

worin

$R^{1b}$ ein Wasserstoffatom oder einen Alkylrest mit 1 bis 4 Kohlenstoffatomen,

$R^{2b}$ die Gruppe $-CO-C_{n^b}H_{2n^b}-R^{3b}$, und

$R^{3b}$ ein Halogenatom darstellt,

$R^{6b}$, $R^{7b}$ und $R^{8b}$ gleich oder verschieden sind und Wasserstoff oder Alkyl mit 1 bis 4 Kohlenstoffatomen bedeuten und

$n^b$ 1 oder 2 darstellt,

und ihre Säureadditionssalze mit anorganischen und organischen Säuren.

5. Tricyclische Pyrrole der allgemeinen Formel $I^b$ nach Anspruch 4, in denen $R^{1b}$ Wasserstoff bedeutet, $R^{2b}$ die in Anspruch 4 angegebene Bedeutung hat, $R^{3b}$ Chlor oder Brom darstellt, $R^{6b}$ Methyl, $R^{7b}$ Wasserstoff, $R^{8b}$ Methyl und $n^b$ 1 oder 2 darstellt, und ihre Säureadditionssalze.

6. Tricyclische Pyrrole nach Anspruch 1, gekennzeichnet durch die allgemeine Formel $I^c$

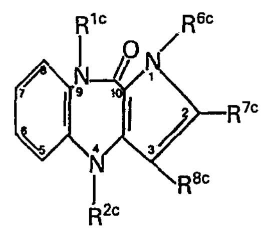

($I^c$)

worin

$R^{1c}$ ein Wasserstoffatom oder einen Alkylrest mit 1 bis 4 Kohlenstoffatomen und

$R^{2c}$ ein Wasserstoffatom darstellt und

$R^{6c}$, $R^{7c}$ und $R^{8c}$ gleich oder verschieden sind und Wasserstoff oder Alkyl mit 1 bis 4 Kohlenstoffatomen bedeuten,

und ihre Säureadditionssalze mit anorganischen und organischen Säuren.

7. Tricyclische Pyrrole der allgemeinen Formel $I^c$ nach Anspruch 6, in denen $R^{1c}$ Wasserstoff bedeutet, $R^{2c}$ die in Anspruch 6 angegebene Bedeutung hat, $R^{6c}$ Methyl, $R^{7c}$ Wasserstoff und $R^{8c}$ Methyl bedeutet, und ihre Säureadditionssalze.

8. 1,3-Dimethyl-4-pyrrolidinoacetyl-1,4,9,10-tetrahydro-pyrrolo[3.2-b][1.5]benzodiazepin-10-on und seine Salze.

9. Verfahren zur Herstellung tricyclischer Pyrrole der allgemeinen Formel I nach Anspruch 1, sowie ihrer Säureadditionssalze mit anorganischen und organischen Säuren, dadurch gekennzeichnet,

dass man Verbindungen der allgemeinen Formel VI

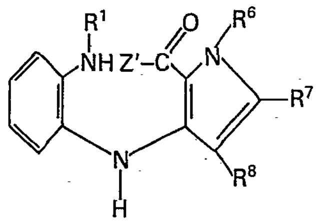

(VI)

worin $R^1$, $R^6$, $R^7$ und $R^8$ die in Anspruch 1 angegebenen Bedeutungen haben und Z' eine geeignete Fluchtgruppe (= Abgangsgruppe) darstellt, cyclisiert und gegebenenfalls anschliessend bei den erhaltenen Tricyclen IV,

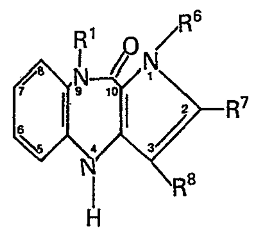

(IV)

worin $R^1$, $R^6$, $R^7$ und $R^8$ die in Anspruch 1 angegebenen Bedeutungen haben, in 4-Position den Rest $-CO-C_nH_{2n}-R^3$, worin $R^3$ und n die in Anspruch 1 angegebene Bedeutung haben, einführt, und dass man erhaltene Basen gegebenenfalls anschliessend in ihre Säureadditionssalze bzw. erhaltene Säureadditionssalze gegegebenenfalls anschliessend in die freien Basen überführt.

10. Arzneimittel enthaltend eine oder mehrere tricyclische Pyrrole der allgemeinen Formel $I^a$ nach Anspruch 2 und/oder 3 und/oder ihre pharmakologisch verträglichen Säureadditionssalze.

11. Tricyclische Pyrrole der allgemeinen Formel $I^a$ nach Anspruch 2 oder 3 und ihre pharmakologisch verträglichen Säureadditionssalze zur Behandlung und Prophylaxe von Krankheiten, die auf Erkrankungen des Magens und Darms beruhen.

**Patentansprüche für den Vertragsstaat AT**

1. Verfahren zur Herstellung tricyclischer Pyrrole der allgemeinen Formel I,

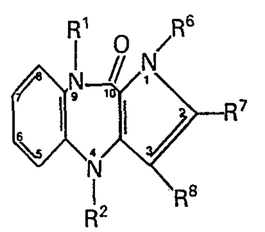

(I)

worin

$R^1$ ein Wasserstoffatom oder einen Alkylrest mit 1 bis 4 Kohlenstoffatomen,

$R^2$ die Gruppe $-CO-C_nH_{2n}-R^3$ oder ein Wasserstoffatom,

$R^3$ ein Halogenatom oder die Gruppe $-N(R^4)R^5$,

$R^4$ ein Wasserstoffatom, einen Alkylrest mit 1 bis 4 Kohlenstoffatomen oder einen Alkenylrest mit 3 bis 5 Kohlenstoffatomen und

$R^5$ einen Alkylrest mit 1 bis 4 Kohlenstoffatomen, der gegebenenfalls durch eine Dialkylaminogruppe mit 1 bis 4 Kohlenstoffatomen in jedem Alkylrest substituiert ist, oder einen Alkenylrest mit 3 bis 5 Kohlenstoffatomen bedeutet, oder

$R^4$ und $R^5$ gemeinsam und unter Einschluss des Stickstoffatoms, an das sie gebunden sind, einen Pyrrolidino-, Piperidino-, Morpholino-, Perhydroazepino- oder einen gegebenenfalls in 4-Position durch eine Methyl-, Ethyl- oder Benzylgruppe substituierten 1-Piperazinylrest oder einen gegebenenfalls in 4-Position durch eine Methylgruppe substituierten 1-Homo-piperazinylrest bedeuten,

$R^6$, $R^7$ und $R^8$ gleich oder verschieden sind und Wasserstoff oder Alkyl mit 1 bis 4 Kohlenstoffatomen bedeuten und

n 1 oder 2 darstellt,

sowie ihre Säureadditionssalze mit anorganischen und organischen Säuren, dadurch gekennzeichnet, dass man Verbindungen der allgemeinen Formel VI,

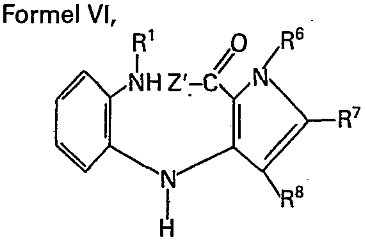

(VI)

worin $R^1$, $R^6$, $R^7$ und $R^8$ die oben angegebenen Bedeutungen haben und Z' eine geeignete Fluchtgruppe (= Abgangsgruppe) darstellt, cyclisiert und gegebenenfalls anschliessend bei den erhaltenen Tricyclen IV,

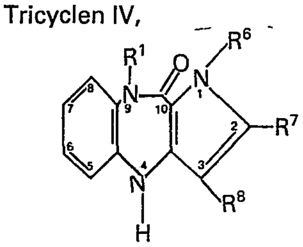

(IV)

worin $R^1$, $R^6$, $R^7$ und $R^8$ die oben angegebenen Bedeutungen haben, in 4-Position den Rest $-CO-C_nH_{2n}-R^3$, worin $R^3$ und n die oben angegebene Bedeutung haben, einführt, und dass man erhaltene Basen gegebenenfalls anschliessend in ihre Säureadditionssalze bzw. erhaltene Säureadditionssalze gegebenenfalls anschliessend in die freien Basen überführt.

2. Verfahren nach Anspruch 1 zur Herstellung tricyclischer Pyrrole der allgemeinen Formel I, worin $R^2$ die Gruppe $-CO-C_nH_{2n}-R^3$ und $R^3$ die Gruppe $-N(R^4)R^5$ darstellt, und ihrer Salze, dadurch gekennzeichnet, dass man in die Tricyclen IV

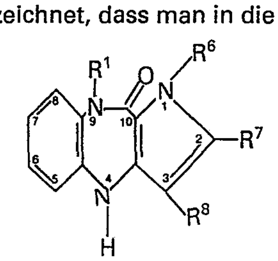

(IV)

den Rest $-CO-C_nH_{2n}-R^3$ (mit $R^3 = -N(R^4)R^5$) in einem Schnitt [Umsetzung von IV mit Verbindungen III'

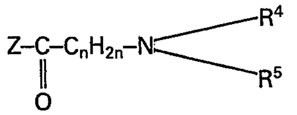

(III')]

oder in zwei Schritten [Umsetzung von IV mit Verbindungen V

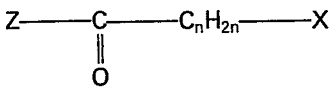

(V)

und anschliessende Umsetzung des erhaltenen Zwischenproduktes II

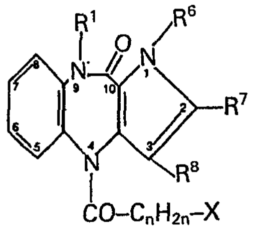

(II)

mit Aminen III

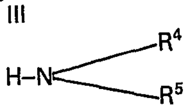

(III)]

einführt und gegebenenfalls anschliessend erhaltene Basen in die Säureadditionssalze oder erhaltene Säureadditionssalze in die Basen überführt, wobei Z eine Fluchtgruppe und X ein Halogenatom darstellt und $R^1$, $R^4$, $R^5$, $R^6$, $R^7$, $R^8$ und n die in Anspruch 1 angegebene Bedeutung haben.

3. Verfahren nach Anspruch 2, dadurch gekennzeichnet, dass $R^1$ Wasserstoff bedeutet, $R^2$ und $R^3$ die in Anspruch 2 angegebene Bedeutung haben, $R^4$ Wasserstoff oder einen Alkylrest mit 1 bis 4 Kohlenstoffatomen bedeutet, $R^5$ einen Alkylrest mit 1 bis 4 Kohlenstoffatomen bedeutet, der gegebenenfalls endständig durch eine Dimethylaminogruppe substituiert ist, oder $R^4$ und $R^5$ gemeinsam eine Pyrrolidino-, Piperidino-, Morpholino-, Piperazino- oder 4-Methyl-1-piperazinylgruppe bedeuten, $R^6$ Methyl, $R^7$ Wasserstoff, $R^8$ Methyl bedeutet und n 1 oder 2 darstellt, Z eine Fluchtgruppe bedeutet und X Chlor oder Brom darstellt.

4. Verfahren nach Anspruch 1 zur Herstellung tricyclischer Pyrrole der allgemeinen Formel I, worin $R^2$ die Gruppe $-CO-C_nH_{2n}-R^3$ und $R^3$ ein Halogenatom darstellt, und ihrer Salze, dadurch gekennzeichnet, dass man in die Tricyclen IV

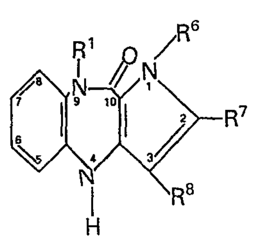

(IV)

den Rest $-CO-C_nH_{2n}-R^3$ (mit $R^3$ = Halogen) durch Umsetzung von IV mit Verbindungen V

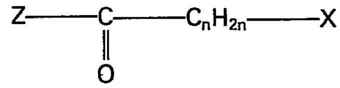

(V)

einführt und gegebenenfalls anschliessend erhaltene Basen in die Säureadditionssalze oder erhaltene Säureadditionssalze in die Basen überführt, wobei Z eine Fluchtgruppe und X ein Halogenatom darstellt und $R^1$, $R^6$, $R^7$, $R^8$ und n die in Anspruch 1 angegebene Bedeutung haben.

5. Verfahren nach Anspruch 4, dadurch gekennzeichnet, dass $R^1$ ein Wasserstoffatom darstellt, $R^2$ die in Anspruch 4 angegebene Bedeutung hat, $R^3$ Chlor oder Brom darstellt, $R^6$ Methyl, $R^7$ Wasserstoff, $R^8$ Methyl und n 1 oder 2 bedeutet, Z eine Fluchtgruppe und X Chlor oder Brom darstellt.

6. Verfahren nach Anspruch 1 zur Herstellung tricyclischer Pyrrole der allgemeinen Formel I, worin $R^2$ ein Wasserstoffatom darstellt, und ihrer Salze, dadurch gekennzeichnet, dass man Verbindungen der allgemeinen Formel VI

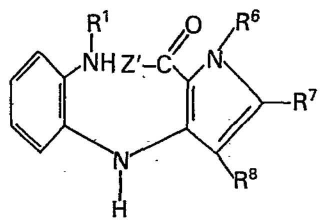

(VI)

cyclisiert, und gegebenenfalls anschliessend erhaltene Basen in die Säureadditonssalze oder erhaltene Säureadditionssalze in die Basen überführt, wobei Z' eine Fluchtgruppe darstellt und $R^1$, $R^6$, $R^7$ und $R^8$ die in Anspruch 1 angegebene Bedeutung haben.

7. Verfahren nach Anspruch 6, dadurch gekennzeichnet, dass $R^1$ Wasserstoff, $R^6$ Methyl, $R^7$ Wasserstoff und $R^8$ Methyl bedeutet und Z' eine Fluchtgruppe darstellt.

8. Verfahren nach Anspruch 2, 3, 4 oder 5, dadurch gekennzeichnet, dass die Fluchtgruppe Z in den Verbindungen V ein Halogenatom darstellt.

9. Verfahren nach Anspruch 2 oder 3, dadurch gekennzeichnet, dass die Fluchtgruppe Z in den Verbindungen III' zusammen mit der Carbonylgruppe, an die sie gebunden ist, ein reaktives Carbonsäureanhydrid bildet.

10. Verfahren nach Anspruch 6 oder 7, dadurch gekennzeichnet, dass die Fluchtgruppe Z' in den Verbindungen VI eine Hydroxy-, Alkoxy- oder Aminogruppe darstellt.

11. Verfahren nach Anspruch 1, dadurch gekennzeichnet, dass 1,3-Dimethyl-4-pyrrolidinoacetyl-1,4,9,10-tetrahydro-pyrrolo[3.2-b][1.5]benzodiazepin-10-on oder ein Salz davon hergestellt wird.

**Claims for the contracting states: BE, CH, DE, FR, GB, IT, LI, LU, NL, SE**

1. Tricyclic pyrroles of the general formula I

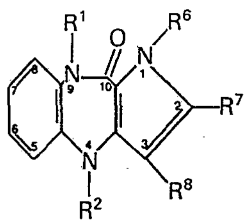

(I)

wherein

$R^1$ denotes a hydrogen atom or an alkyl radical with 1 to 4 carbon atoms,

$R^2$ denotes the group $-CO-C_nH_{2n}-R^3$ or a hydrogen atom,

$R^3$ denotes a halogen atom or the group $-N(R^4)R^5$,

$R^4$ denotes a hydrogen atom, an alkyl radical with 1 to 4 carbon atoms or an alkenyl radical with 3 to 5 carbon atoms and

$R^5$ denotes an alkyl radical which has 1 to 4 carbon atoms and is optionally substituted by a dialkylamino group with 1 to 4 carbon atoms, in each alkyl radical, or denotes an alkenyl radical with 3 to 5 carbon atoms, or

$R^4$ and $R^5$ together, with the inclusion of the nitrogen atom to which they are bonded, denote a pyrrolidino, piperidino, morpholino or perhydroazepino ring, a 1-piperazinyl radical which is optionally substituted in the 4-position by a methyl, ethyl or benzyl group, or a 1-homo-piperazinyl radical which is optionally substituted in the 4-position by a methyl group,

Homo-piperazinylrest bedeuten,

$R^6$, $R^7$ and $R^8$ are identical or different and denote hydrogen or alkyl with 1 to 4 carbon atoms and

n represents 1 or 2,

and their acid addition salts with inorganic and organic acids.

2. Tricyclic pyrroles according to claim 1, characterized by the general formula $I^a$

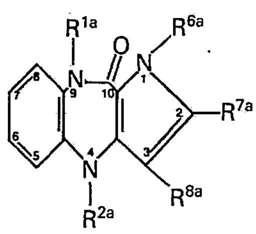

($I^a$)

wherein

$R^{1a}$ represents a hydrogen atom or an alkyl radical with 1 to 4 carbon atoms,

$R^{2a}$ represents the group $-CO-C_{n^a}H_{2n^a}-R^{3a}$,

$R^{3a}$ represents the group $-N(R^{4a})R^{5a}$,

$R^{4a}$ represents a hydrogen atom, an alkyl radical with 1 to 4 carbon atoms or an alkenyl radical with 3 to 5 carbon atoms and

$R^{5a}$ represents an alkyl radical which has 1 to 4 carbon atoms and is optionally substituted by a dialkylamino group with 1 to 4 carbon atoms in each alkyl radical or represents an alkenyl radical with 3 to 5 carbon atoms, or

$R^{4a}$ and $R^{5a}$ together, with the inclusion of the nitrogen atom to which they are bonded, denote a pyrrolidino, piperidino, morpholino or perhydroazepino ring, a 1-piperazinyl radical which is optionally substituted in the 4-position by a methyl, ethyl or benzyl group, or a 1-homo-piperazinyl radical which is optionally substituted in the 4-position by a methyl group,

$R^{6a}$, $R^{7a}$ and $R^{8a}$ are identical or different and denote hydrogen or alkyl with 1 to 4 carbon atoms and

$n^a$ represents 1 or 2,

and their acid addition salts with inorganic and organic acids.

3. Tricyclic pyrroles of the general formula $I^a$ according to claim 2, wherein $R^{1a}$ denotes a hydrogen atom, $R^{2a}$ and $R^{3a}$ have the meaning given in claim 2, $R^{4a}$ denotes a hydrogen atom or an alkyl radical with 1 to 4 carbon atoms, $R^{5a}$ denotes an alkyl radical with 1 to 4 carbon atoms which is optionally terminally substituted by a dimethylamino group, or $R^{4a}$ and $R^{5a}$ together denote a pyrrolidino, piperidino, morpholino or piperazino ring or a 4-methyl-1-piperazinyl group, $R^{6a}$ denotes a methyl group, $R^{7a}$ denotes a hydrogen atom, $R^{8a}$ denotes a methyl group and $n^a$ represents 1 or 2, and their pharmacologically compatible acid addition salts with inorganic and organic acids.

4. Tricyclic pyrroles according to claim 1, characterized by the general formula $I^b$

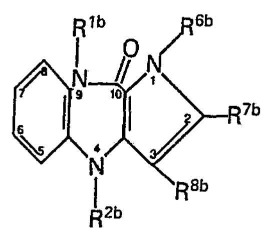

($I^b$)

wherein

$R^{1b}$ represents a hyrogen atom or an alkyl radical with 1 to 4 carbon atoms,

$R^{2b}$ represents the group $-CO-C_{n^b}H_{2n^b}-R^{3b}$ and

$R^{3b}$ represents a halogen atom,

$R^{6b}$, $R^{7b}$ and $R^{8b}$ are identical or different and denote hydrogen or alkyl with 1 to 4 carbon atoms and

$n^b$ represents 1 or 2,

and their acid addition salts with inorganic and organic acids.

5. Tricyclic pyrroles of the general formula $I^b$ according to claim 4, wherein $R^{1b}$ denotes a hydrogen atom, $R^{2b}$ has the meaning given in claim 4, $R^{3b}$ represents chlorine or bromine, $R^{6b}$ denotes a methyl group, $R^{7b}$ denotes a hydrogen atom, $R^{8b}$ denotes a methyl group and $n^b$ represents 1 or 2, and their acid addition salts.

6. Tricyclic pyrroles according to claim 1, characterized by the general formula $I^c$

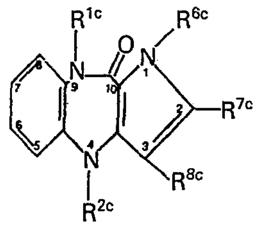

($I^c$)

wherein

$R^{1c}$ represents a hydrogen atom or an alkyl radical with 1 to 4 carbon atoms and

$R^{2c}$ represents a hydrogen atom and

$R^{6c}$, $R^{7c}$ and $R^{8c}$ are identical or different and denote hydrogen or alkyl with 1 to 4 carbon atoms,

and their acid addition salts with inorganic and organic acids,

7. Tricyclic pyrroles of the general formula $I^c$ according to claim 6, in which $R^{1c}$ denotes a hydrogen atom, $R^{2c}$ has the meaning given in claim 6, $R^{6c}$ denotes a methyl group, $R^{7c}$ denotes a hydrogen atom and $R^{8c}$ denotes a methyl group and their acid additions salts.

8. 1,3-Dimethyl-4-pyrrolidinoacetyl-1,4,9,10-tetrahydro-pyrrolo[3.2-b][1.5]benzodiazepin-10-one and its salts.

9. Process for the preparation of tricyclic pyrroles of the general formula I according to claim 1, and their acid addition salts with inorganic and organic acids, characterized in that compounds of the general formula VI

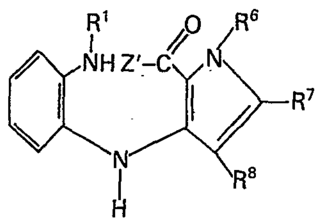

(VI)

wherein $R^1$, $R^6$, $R^7$ and $R^8$ have the meanings given in claim 1 and Z' denotes a suitable leaving group, are subjected to cyclisation, and that optionally in the 4-position of the obtained tricyclic compounds IV

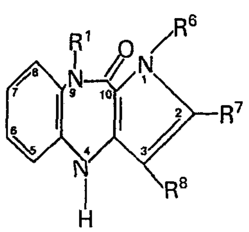

(IV)

wherein $R^1$, $R^6$, $R^7$ and $R^8$ have the meanings given in claim 1, the radical $-CO-C_nH_{2n}-R^3$, wherein $R^3$ and n have the meanings given in claim 1, is subsequently introduced, and that obtained bases are optionally subsequently converted into their acid addition salts or that obtained acid addition salts are subsequently converted into the free bases.

10. Medicaments containing one or more tricyclic pyrroles of the general formula $I^a$ according to claim 2 and/or 3 and/or their pharmacologically acceptable acid addition salts.

11. Tricyclic pyrroles of the general formula $I^a$ of the general formula $I^a$ according to claim 2 or 3 and their pharmacologically acceptable acid addition salts for use in the treatment and prophylaxis of diseases of the stomach or intestine.

**Claims for the contracting state: AT**

1. Process for the preparation of tricyclic pyrroles of the general formula I

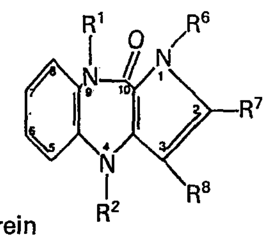

(I)

wherein

$R^1$ denotes a hydrogen atom or an alkyl radical with 1 to 4 carbon atoms,

$R^2$ denotes the group $-CO-C_nH_{2n}-R^3$ or a hydrogen atom,

$R^3$ denotes a halogen atom or the group $-N(R^4)R^5$,

$R^4$ denotes a hydrogen atom, an alkyl radical with 1 to 4 carbon atoms or an alkenyl radical with 3 to 5 carbon atoms and

$R^5$ denotes an alkyl radical which has 1 to 4 carbon atoms and is optionally substituted by a dialkylamino group with 1 to 4 carbon atoms, in each alkyl radical, or denotes an alkenyl radical with 3 to 5 carbon atoms, or

$R^4$ and $R^5$ together, with the inclusion of the nitrogen atom to which they are bonded, denote a pyrrolidino, piperidino, morpholino or perhydroazepino ring, a 1-piperazinyl radical which is optionally substituted in the 4-position by a methyl, ethyl or benzyl group, or a 1-homo-piperazinyl radical which is optionally substituted in the 4-position by a methyl group,

$R^6$, $R^7$ and $R^8$ are identical or different and denote hydrogen or alkyl with 1 to 4 carbon atoms and

n represents 1 or 2,

and their acid addition salts with inorganic and organic acids, characterized in that general compounds of the formula VI

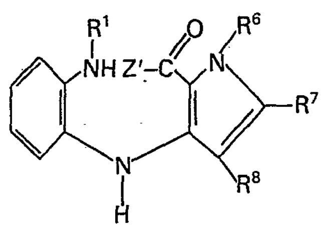

(VI)

wherein $R^1$, $R^6$, $R^7$ and $R^8$ have the meanings given above and Z' denotes a suitable leaving group, are subjected to cyclisation, and that optionally in the

4-position of the obtained tricyclic compounds IV

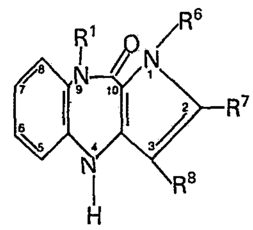

(IV)

wherein $R^1$, $R^6$, $R^7$ and $R^8$ have the meanings given above, the radical $-CO-C_nH_{2n}-R^3$, wherein $R^3$ and n have the meanings given above, is subsequently introduced, and that obtained bases are optionally subsequently converted into their acid addition salts or that obtained acid addition salts are subsequently converted into the free bases.

2. Process according to claim 1 for the preparation of tricyclic pyrroles of the general formula I, wherein $R^2$ denotes the group $-CO_nH_{2n}-R^3$ and $R^3$ the denotes the group $-N(R^4)R^5$ and their salts, characterized in that the introduction of the radical $-CO-C_nH_{2n}-R^3$ (with $R^3 = -N(R^4)R^5$) is carried out in one step by reacting compound IV

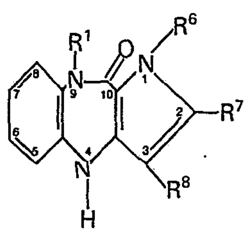

(IV)

with compound III'

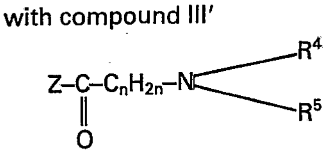

(III')

or in two steps by reaction of compound IV with compound V

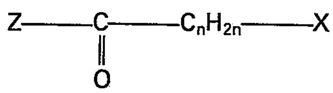

(V)

and subsequent reaction of the resulting product II

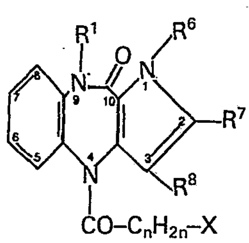

(II)

with amines III

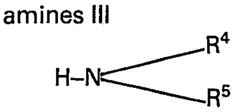

(III)

and that obtained free bases are optionally subsequently converted into the acid addition salts or that obtained acid addition salts are optionally subsequently converted into the free bases, wherein Z represents a leaving group and X represents a halogen atom and $R^1$, $R^4$, $R^5$, $R^6$, $R^7$, $R^8$ and n have the meanings given in claim 1.

3. Process according to claim 2, characterized in that $R^1$ denotes hydrogen, $R^2$ and $R^3$ have the meanings given in claim 2, $R^4$ denotes a hydrogen atom or an alkyl radical with 1 to 4 carbon atoms, $R^5$ denotes an alkyl radical with 1 to 4 carbon atoms, which is optionally terminally substituted by a dimethylamino group, or $R^4$ and $R^5$ together represents a pyrrolidino, piperidino, morpholino, piperazino or a 4-methyl-1-piperazinyl group, $R^6$ represents a methyl group, $R^7$ denotes a hydrogen atom, $R^8$ denotes a methyl group and n represents 1 or 2, Z represents a leaving group and X denotes chlorine or bromine.

4. Process according to claim 1 for the preparation of tricyclic pyrroles of the general formula I, wherein $R^2$ denotes the radical $-CO-C_nH_{2n}-R^3$ and $R^3$ represents a halogen atom and their salts characterized in that the introduction of the radical $-CO-C_nH_{2n}-R^3$ (with $R^3$ = halogen) is carried out by reacting tricyclic compounds IV

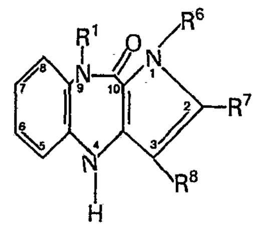

(IV)

with compound V

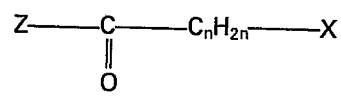

(V)

and that obtained free bases are optionally subsequently converted into the acid addition salts or that obtained acid addition salts are optionally subsequently converted into the free bases, wherein Z represents a leaving group and X represents a halogen atom and $R^1$, $R^6$, $R^7$, $R^8$ and n have the meanings given in claim 1.

5. Process according to claim 4, characterized in that $R^1$ denotes a hydrogen atom, $R^2$ has the meaning given in claim 4, $R^3$ denotes chlorine or bromine, $R^6$ denotes a methyl group, $R^7$ denotes a hydrogen atom, $R^8$ denotes a methyl group and n represents 1 or 2, Z denotes a leaving group and X denotes chlorine or bromine.

6. Process according to claim 1 for the preparation of tricyclic pyrroles of the general formula I, wherein $R^2$ denotes a hydrogen atom, and their salts, characterized in, that compounds of the general formula VI

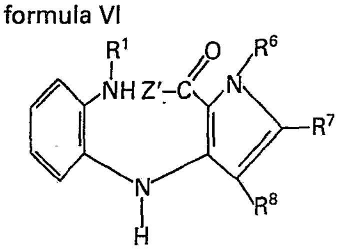

(VI)

are subjected to cyclization and that obtained free bases are optionally subsequently converted into the acid addition salts or that obtained acid addition salts are optionally subsequently converted into the free bases, wherein Z' represents a leaving group and $R^1$, $R^6$, $R^7$ and $R^8$ have the meanings given in claim 1.

7. Process according to claim 6, characterized in, that $R^1$ denotes a hydrogen atom, $R^6$ denotes a methyl group, $R^7$ denotes a hydrogen atom and $R^8$ denotes a methyl group and Z' represents a leaving group.

8. Processes according to claims 2, 3, 4 or 5, characterized in, that the leaving group Z in the compounds V denotes a halogen atom.

9. Processes according to claims 2 or 3, characterized in, that in compounds III' the leaving group Z together with the carbonyl group to which it is bonded, forms a reactive carboxylic acid derivate.

10. Processes according to claims 6 or 7, characterized in, that the leaving group Z' in the compounds VI denotes a hydroxy, alkoxy or amino group.

11. Process according to claim 1, characterized in, that 1,3-dimethyl-4-pyrrolidinoacetyl-1,4,9,10-tetrahydropyrrolo[3.2-b][1.5]benzodiazepin-10-one or a salt thereof is prepared.

**Revendications pour les Etats contractants: BE, CH, DE, FR, GB, IT, LI, LU, NL, SE**

1. Pyrroles tricycliques de formule générale I:

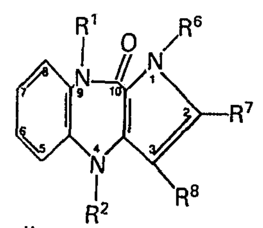

(I)

dans laquelle

$R^1$ est un atome d'hydrogène ou un radical alkyle ayant de 1 à 4 atomes de carbone,

$R^2$ est le groupe $-CO-C_nH_{2n}-R^3$ ou un atome d'hydrogène,

$R^3$ est un atome d'halogène ou le groupe $-N(R^4)R^5$,

$R^4$ est un atome d'hydrogène, un radical alkyle ayant de 1 à 4 atomes de carbone ou un radical alcényle ayant de 3 à 5 atomes de carbone et

$R^5$ est un radical alkyle ayant de 1 à 4 atomes de carbone, qui est éventuellement substitué par un groupe dialkylamino ayant de 1 à 4 atomes de carbone dans chaque radical alkyle, ou un radical alcényle ayant de 3 à 5 atomes de carbone, ou

$R^4$ et $R^5$ forment ensemble et en incluant l'atome d'azote sur lequel ils sont fixés un radical pyrrolidino, pipéridino, morpholino, perhydroazépino ou 1-pipérazinyle substitué éventuellement en position 4 par un groupe méthyle, éthyle ou benzyle, ou bien un radical 1-homo-pipérazinyle éventuellement substitué en position 4 par un groupe méthyle,

$R^6$, $R^7$ et $R^8$ sont identiques ou différents et sont de l'hydrogène ou un alkyle ayant de 1 à 4 atomes de carbone et

n est égal à 1 ou 2,

et leurs sels d'addition avec des acides inorganiques et organiques.

2. Pyrroles tricycliques suivant la revendication 1, caractérisés par la formule générale $I^a$:

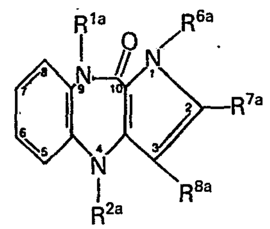

($I^a$)

dans laquelle

$R^{1a}$ est un atome d'hydrogène ou un radical alkyle ayant de 1 à 4 atomes de carbone,

$R^{2a}$ est le groupe $-CO-C_{n^a}H_{2n^a}-R^{3a}$,

$R^{3a}$ est le groupe $-N(R^{4a})R^{5a}$,

$R^{4a}$ est un atome d'hydrogène, un radical alkyle ayant de 1 à 4 atomes de carbone ou un radical alcényle ayant de 3 à 5 atomes de carbone et

$R^{5a}$ est un radical alkyle ayant de 1 à 4 atomes de carbone, qui est éventuellement substitué par un groupe dialkylamino ayant de 1 à 4 atomes de carbone dans chaque radical alkyle, ou un radical alcényle ayant de 3 à 5 atomes de carbone, ou

$R^{4a}$ et $R^{5a}$ forment ensemble et en incluant l'atome d'azote sur lequel ils sont fixés un radical pyrrolidino, pipéridino, morpholino, perhydroazépino ou un radical 1-pipérazinyle éventuellement substitué en position 4 par un groupe méthyle, éthyle ou benzyle, ou bien un radical 1-homo-pipérazinyle éventuellement substitué en position 4 par un groupe méthyle,

$R^{6a}$, $R^{7a}$ et $R^{8a}$ sont identiques ou différents et sont de l'hydrogène ou un alkyle ayant de 1 à 4 atomes de carbone et

$n^a$ est égal à 1 ou 2,

et leurs sels d'addition avec des acides inorganiques et organiques.

3. Pyrroles tricycliques de formule générale $I^a$ suivant la revendication 2, dans laquelle $R^{1a}$ est de l'hydrogène, $R^{2a}$ et $R^{3a}$ ont la signification indiquée dans la revendication 2, $R^{4a}$ est de l'hydrogène ou un radical alkyle ayant de 1 à 4 atomes de carbone, $R^{5a}$ est un radical alkyle ayant de 1 à 4 atomes de carbone, qui est substitué éventuellement en position terminale par un groupe diméthylamino, ou bien $R^{4a}$ et $R^{5a}$ forment ensemble un groupe pyrrolidino, pipéridino, morpholino, pipérazino ou 4-méthyl-1-pipérazinyle, $R^{6a}$ est un méthyle, $R^{7a}$ est de l'hydrogène, $R^{8a}$ est un méthyle et $n^a$ est égal à 1 ou 2, et leurs sels d'addition pharmaceutiquement acceptables avec les acides inorganiques et organiques.

4. Pyrroles tricycliques suivant la revendication 1, caractérisés par la formule générale $I^b$:

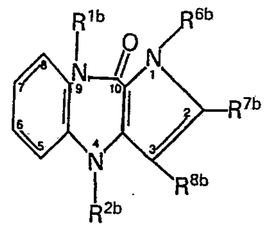

($I^b$)

dans laquelle:

$R^{1b}$ est un atome d'hydrogène ou un radical alkyle ayant de 1 à 4 atomes de carbone,

$R^{2b}$ est le groupe $-CO-C_{n^b}H_{2n^b}-R^{3b}$ et

$R^{3b}$ est un atome d'halogène,

$R^{6b}$, $R^{7b}$ et $R^{8b}$ sont identiques ou différents et sont de l'hydrogène ou un alkyle ayant de 1 à 4 atomes de carbone, et

$n^b$ est égal à 1 ou 2,

et leurs sels d'addition avec des acides inorganiques et organiques.

5. Pyrroles tricycliques de formule générale $I^b$ suivant la revendication 4, dans laquelle $R^{1b}$ est de l'hydrogène, $R^{2b}$ a la signification indiquée dans la revendication 4, $R^{3b}$ est du chlore ou du brome, $R^{6b}$ est un méthyle, $R^{7b}$ est de l'hydrogène, $R^{8b}$ est un méthyle et $n^b$ est égal à 1 ou 2, et leurs sels d'addition avec des acides.

6. Pyrroles tricycliques suivant la revendication 1, caractérisés par la formule générale $I^c$:

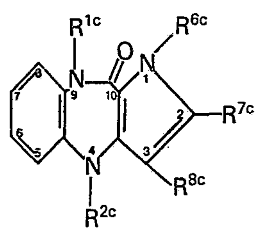

($I^c$)

dans laquelle:

$R^{1c}$ est un atome d'hydrogène ou un radical alkyle ayant de 1 à 4 atoms de carbone et

$R^{2c}$ est un atome d'hydrogène et

$R^{6c}$, $R^{7c}$ et $R^{8c}$ sont identiques ou différents et sont de l'hydrogène ou un alkyle ayant de 1 à 4 atomes de carbone,

et leurs sels d'addition avec des acides inorganiques et organiques.

7. Pyrroles tricycliques de formule générale $I^c$ suivant la revendication 6, dans laquelle $R^{1c}$ est de l'hydrogène, $R^{2c}$ a la signification indiquée dans la revendication 6, $R^{6c}$ est un méthyle, $R^{7c}$ est de l'hydrogène et $R^{8c}$ est un méthyle et leurs sels d'addition avec des acides.

8. 1,3-Diméthyl-4-pyrrolidinoacétyl-1,4,9,10-tétrahydro-pyrrolo[3.2-b][1.5]benzodiazépin-10-one et ses sels.

9. Procédé pour la préparation de pyrroles tricycliques de formule générale I suivant la revendication 1, ainsi que de leurs sels d'addition avec des acides inorganiques et organiques, caractérisé en ce qu'on effectue la cyclisation de composés de formule générale VI

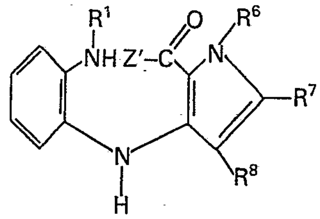

(VI)

dans laquelle $R^1$, $R^6$, $R^7$ et $R^8$ ont la signification indiquée dans la revendication 1 et Z' est un groupe dissociable ou éliminable (groupe séparable) approprié et en ce qu'on introduit éventuellement ensuite dans les tri-cycles obtenus IV

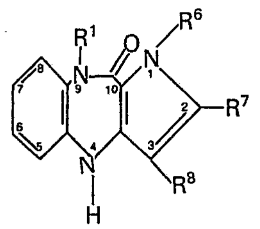

(IV)

dans lesquels $R^1$, $R^6$, $R^7$ et $R^8$ ont les significations indiquées dans la revendication 1, en position 4, le radical $-CO-C_nH_{2n}-R^3$, où $R^3$ et n ont la signification indiquée dans la revendication 1, et en ce qu'on convertit ensuite éventuellement les bases obtenues en leurs sels d'addition avec des acides, ou bien ensuite éventuellement les sels d'addition avec des acides obtenus en bases libres.

10. Médicament contenant un ou plusieurs pyrroles tricycliques de formule générale $I^a$ suivant les revendications 2 et/ou 3 et/ou leurs sels d'addition avec des acides pharmaceutiquement acceptables.

11. Pyrroles tricycliques de formule générale $I^a$ suivant la revendication 2 ou 3 et leurs sels d'addition avec des acides pharmaceutiquement acceptables pour le traitement ou la prophylaxie de maladies qui reposent sur des affections de l'estomac et de l'intestin.

**Revendications pour l'Etat contractant: AT**

1. Procédé pour la préparation de pyrroles tricycliques de formule générale I:

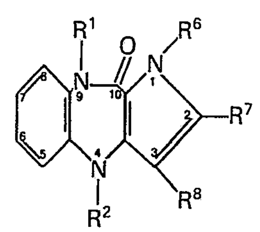

(I)

dans laquelle

$R^1$ est un atome d'hydrogène ou un radical alkyle ayant de 1 à 4 atomes de carbone,

$R^2$ est le groupe $-CO-C_nH_{2n}-R^3$ ou un atome d'hydrogène,

$R^3$ est un atome d'halogène ou le groupe $-N(R^4)R^5$,

$R^4$ est un atome d'hydrogène, un radical alkyle ayant de 1 à 4 atomes de carbone ou un radical alcényle ayant de 3 à 5 atomes de carbone et

$R^5$ est un radical alkyle ayant de 1 à 4 atomes de carbone, qui est éventuellement substitué par un groupe dialkylamino ayant de 1 à 4 atomes de carbone dans chaque radical alkyle, ou un radical alcényle ayant de 3 à 5 atomes de carbone, ou

$R^4$ et $R^5$ forment ensemble et en incluant l'atome d'azote sur lequel ils sont fixés un radical pyrrolidino, pipéridino, morpholino, perhydroazépino ou 1-pipérazinyle substitué éventuellement en position 4 par un groupe méthyle, éthyle ou benzyle, ou bien un radical 1-homo-pipérazinyle éventuellement substitué en position 4 par un groupe méthyle,

$R^6$, $R^7$ et $R^8$ sont identiques ou différents et sont de l'hydrogène ou un alkyle ayant de 1 à 4 atomes de carbone et

n est égal à 1 ou 2,

ainsi que leurs sels d'addition avec des acides inorganiques et organiques, caractérisé en ce qu'on effectue la cyclisation de composés de formule générale VI:

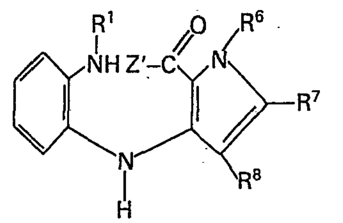

(VI)

dans laquelle $R^1$, $R^6$, $R^7$ et $R^8$ ont la signification indiquée ci-dessus et Z' est un groupe dissociable (groupe séparable) approprié et en ce qu'on introduit éventuellement ensuite dans les tri-cycles obtenus IV

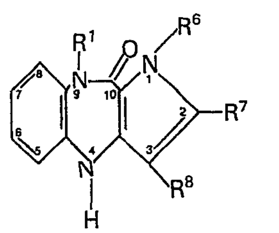

(IV)

dans lesquelle $R^1$, $R^6$, $R^7$ et $R^8$ ont les significations indiquées ci-dessus, en position 4, le radical $-CO-C_nH_{2n}-R^3$, où $R^3$ et n ont la signification indiquée ci-dessus, et en ce qu'on convertit ensuite éventuellement les bases obtenues en leurs sels d'addition avec des acides, ou bien ensuite éventuellement les sels d'addition avec des acides obtenus en bases libres.

2. Procédé suivant la revendication 1 pour la préparation de pyrroles tricycliques de formule générale I dans laquelle $R^2$ est le groupe $-CO-C_nH_{2n}-R^3$ et $R^3$ est le groupe $-N(R^4)R^5$, et de leurs sels, caractérisé en ce qu'on introduit dans les tri-cycles IV

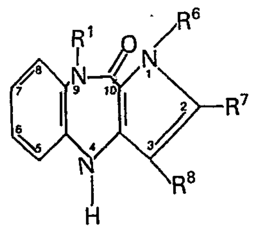

(IV)

le radical $-CO-C_nH_{2n}-R^3$ (où $R^3 = -N(R^4)R^5$) en un stade opératoire [réaction de IV avec des composés III'

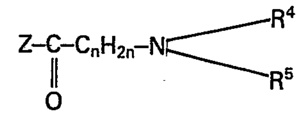

(III')]

ou en deux stades opératoires [réaction de IV avec des composés V

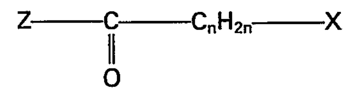

(V)

puis réaction du produit intermédiaire II obtenu

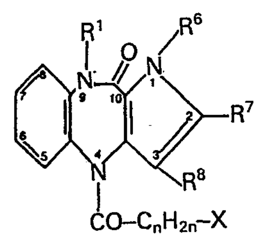

(II)

avec des amines III

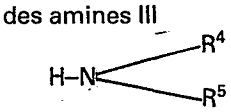

(III)]

puis éventuellement on convertit les bases obtenues en sels d'addition avec des acides ou les sels d'addition avec des acides obtenus en bases, Z étant un groupe dissociable ou éliminable et X est un atome d'halogène, et $R^1$, $R^4$, $R^5$, $R^6$, $R^7$, $R^8$ et n ayant la signification indiquée dans la revendication 1.

3. Procédé suivant la revendication 2, caractérisé en ce que $R^1$ est de l'hydrogène, $R^2$ et $R^3$ ont la signification indiquée dans la revendication 2, $R^4$ est de l'hydrogène ou un radical alkyle ayant de 1 à 4 atomes de carbone, $R^5$ est un radical alkyle ayant de 1 à 4 atomes de carbone, qui est éventuellement substitué en position terminale par un groupe diméthylamino, ou bien $R^4$ et $R^5$ forment ensemble un groupe pyrrolidino, pipéridino, morpholino, pipérazino ou 4-méthyl-1-pipérazinyle, $R^6$ est méthyle, $R^7$ est de l'hydrogène, $R^8$ est un méthyle et n est égal à 1 ou 2, Z est un groupe dissociable et X est du chlore ou du brome.

4. Procédé suivant la revendication 1 pour la préparation de pyrroles tricycliques de formule I dans laquelle $R^2$ est le groupe $-CO-C_nH_{2n}-R^3$ et $R^3$ est un atome d'halogène, et de leurs sels, caractérisé en ce qu'on introduit dans les tri-cycles IV

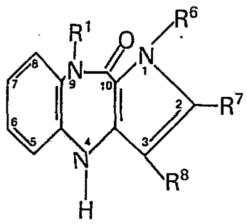

(IV)

le radical $-CO-C_nH_{2n}-R^3$ (où $R^3$ = halogène) par réaction de IV avec des composés V

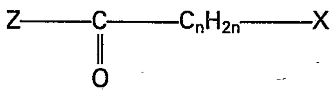

(V)

puis éventuellement on convertit les bases obtenues en sels d'addition avec des acides ou les sels d'addition avec des acides obtenus en bases, Z étant un groupe dissociable et X est un atome d'halogène, et $R^1$, $R^6$, $R^7$, $R^8$ et n ayant la signification indiquée dans la revendication 1.

5. Procédé suivant la revendication 4, caractérisé en ce que $R^1$ est un atome d'hydrogène, $R^2$ a la signification indiquée dans la revendication 4, $R^3$ est du chlore ou du brome, $R^6$ est un méthyle, $R^7$ et de l'hydrogène, $R^8$ est un méthyle et n est égal à 1 ou 2, Z étant un groupe dissociable et X du chlore ou du brome.

6. Procédé suivant la revendication 1 pour la préparation de pyrroles tricycliques de formule générale I dans laquelle $R^2$ est un atome d'hydrogène, et de leurs sels, caractérisé en ce qu'on effectue la cyclisation de composés de formule générale VI.

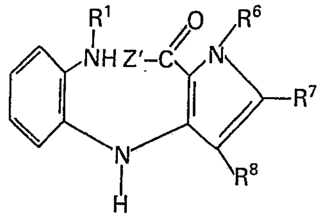

(VI)

puis éventuellement on convertit les bases obtenues en sels d'addition avec des acides ou les sels d'addition avec des acides obtenus en bases, Z' étant un groupe dissociable et $R^1$, $R^6$, $R^7$ et $R^8$ ayant la signification indiquée dans la revendication 1.

7. Procédé suivant la revendication 6, caractérisé en ce que $R^1$ est de l'hydrogène, $R^6$ est un méthyle, $R^7$ est de l'hydrogène et $R^8$ est un méthyle, et Z' est un groupe dissociable.

8. Procédé suivant la revendication 2, 3, 4 ou 5, caractérisé en ce que le groupe dissociable Z dans les composés V est un atome d'halogène.

9. Procédé suivant la revendication 2 ou 3, caractérisé en ce que le groupe dissociable Z dans les composés III' forme conjointement au groupe carbonyle sur lequel il est fixé un anhydride d'acide carboxylique réactif.

10. Procédé suivant la revendication 6 ou 7, caractérisé en ce que le groupe dissociable Z' dans les composés VI est un groupe hydroxy, alcoxy ou amino.

11. Procédé suivant la revendication 1, caractérisé en ce qu'il est appliqué à la préparation de 1,3-diméthyl-6-pyrrolidinoacétyl-1,4,9,10-tétrahydropyrrolo[3.2-b][1.5]benzodiazépin-10-one ou d'un sel de ce composé.